# EUROPEAN PATENT APPLICATION

(11) **EP 4 715 787 A1**
(43) Date of publication of application: **25.03.2026**
(21) Application number: 24807001.3
(22) Date of filing: 24.04.2024
(51) Int. Cl.: G09B 23/28, G09B 9/00, G06T 19/00

(54) **SIMULATOR, SIMULATION METHOD, SIMULATION PROGRAM, AND SIMULATOR SYSTEM**

(30) Priority: 18.05.2023 JP 2023082433
(71) Applicant: Sony Group Corporation, Tokyo 108-0075 (JP)
(72) Inventor: HEREDIA, Saul, Tokyo 108-0075 (JP)
(74) Representative: D Young & Co LLP
(86) International application number: PCT/JP2024/016001
(87) International publication number: WO 2024/237047

(57) **Abstract**

A simulator includes a processing unit including at least one processor. The processing unit simulates at least one of bleeding and smoke release from a biological tissue, based on heating in the biological tissue caused by an energy device, and heat propagation in the biological tissue.

## Description

### Technical Field

The present disclosure relates to a simulator, a simulation method, a simulation program, and a simulator system.

### Background Art

For example, as described in PTL 1, various techniques relating to a simulation of cutting a three-dimensional object are proposed.

### Citation List

### Patent Literature

PTL 1: JP 2003-141566 A

### Summary

### Technical Problem

When a biological tissue is cut during surgery or the like, bleeding may occur. Smoke release may also be caused when a heated surgical tool such as an electric scalpel is used. A simulation including such bleeding or smoke release is not specifically considered in PTL 1.

According to an aspect of the present disclosure, it is possible to simulate a surgery with bleeding and smoke release.

### Solution to Problem

A simulator according to an aspect of the present disclosure includes a processing unit including at least one processor, and the processing unit simulates at least one of bleeding and smoke release from a biological tissue, based on heating in the biological tissue caused by an energy device, and heat propagation in the biological tissue.

A simulation method according to an aspect of the present disclosure includes simulating at least one of bleeding and smoke release from a biological tissue, based on heating in the biological tissue caused by an energy device, and heat propagation in the biological tissue.

A simulation program according to an aspect of the present disclosure causes a computer to execute processing of simulating at least one of bleeding and smoke release from a biological tissue, based on heating in the biological tissue caused by an energy device, and heat propagation in the biological tissue.

A simulator system according to an aspect of the present disclosure includes an inputting apparatus that receives a user operation for moving an energy device, and a display device that displays at least one of bleeding and smoke release from a biological tissue, the bleeding and the smoke release being simulated based on heating in the biological tissue caused by the energy device, and heat propagation in the biological tissue.

### Brief Description of Drawings

[Fig. 1]

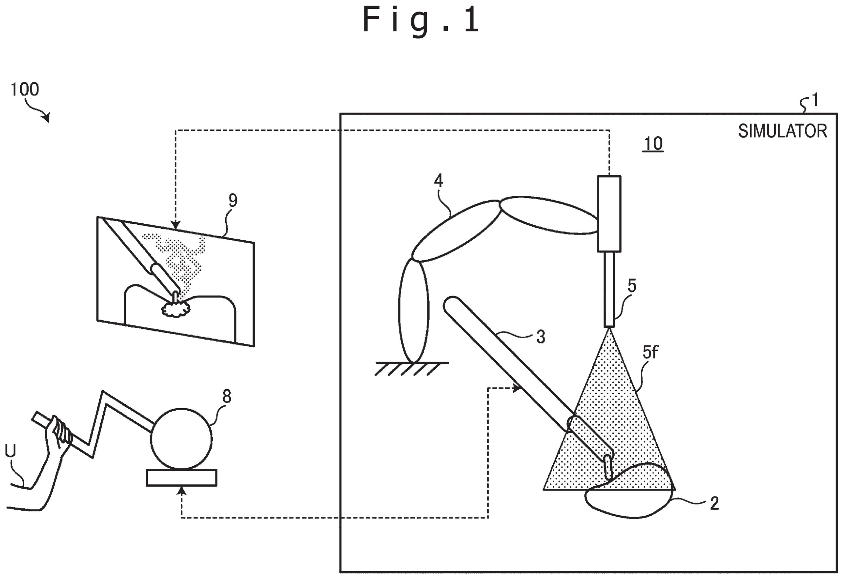

   Fig. 1 is a diagram illustrating an example of a schematic configuration of a simulator system 100 according to an embodiment.
[Fig. 2]

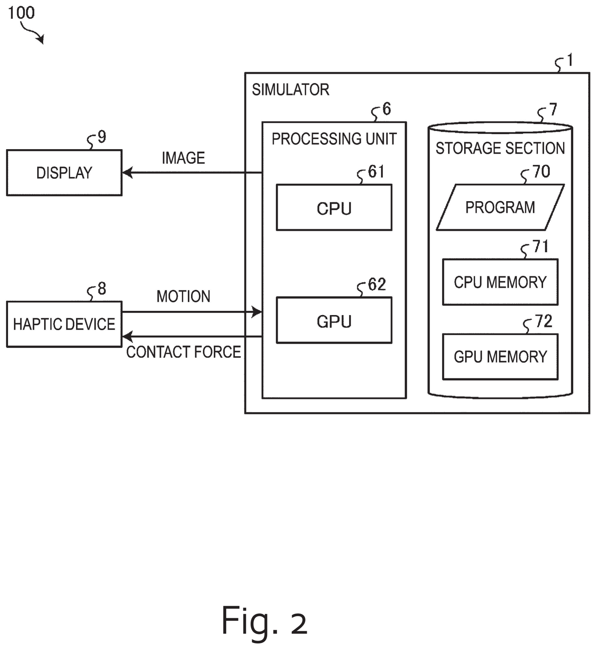

   Fig. 2 is a diagram illustrating an example of a functional block of a simulator 1.
[Fig. 3]

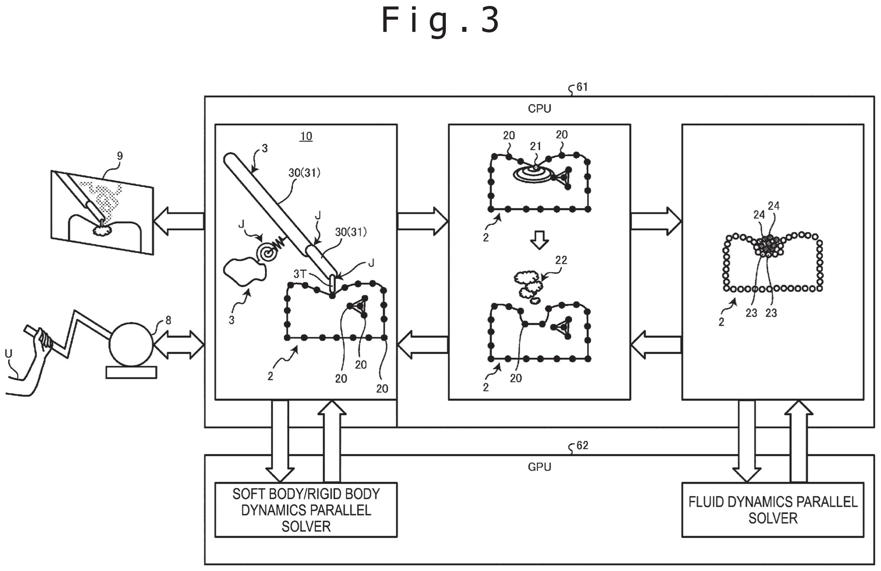

   Fig. 3 is a diagram illustrating an example of roles of a CPU 61 and a GPU 62 in a processing unit 6.
[Fig. 4]

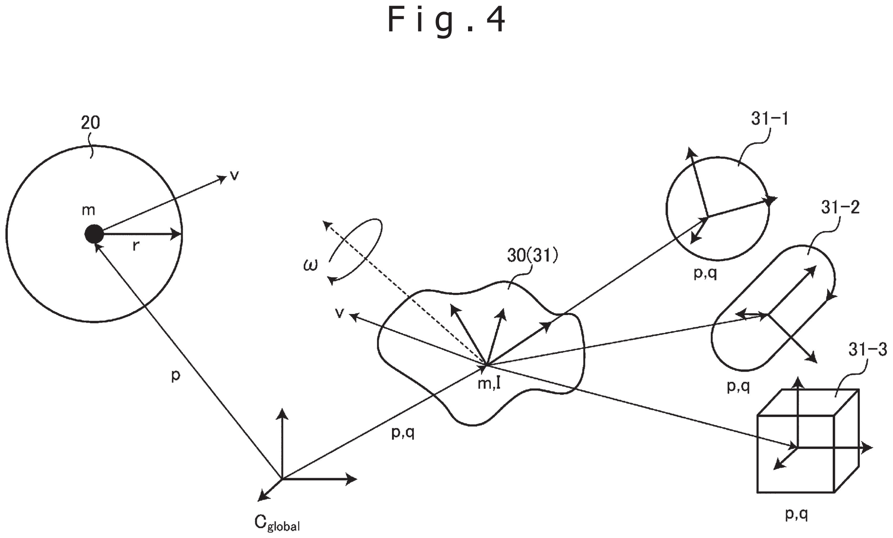

   Fig. 4 is a diagram illustrating an example of a particle 20 and a rigid body 30.
[Fig. 5]

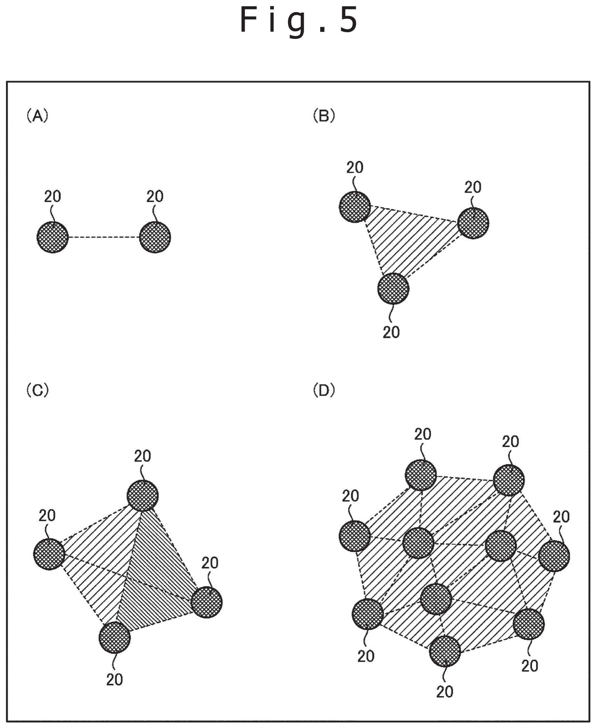

   Fig. 5 is a diagram illustrating examples of constraints.
[Fig. 6]

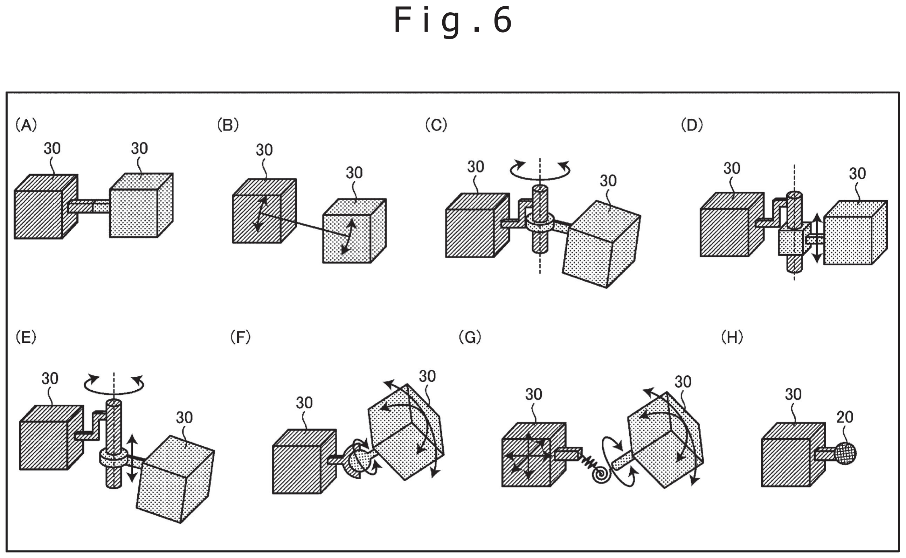

   Fig. 6 is a diagram illustrating examples of constraints.
[Fig. 7]

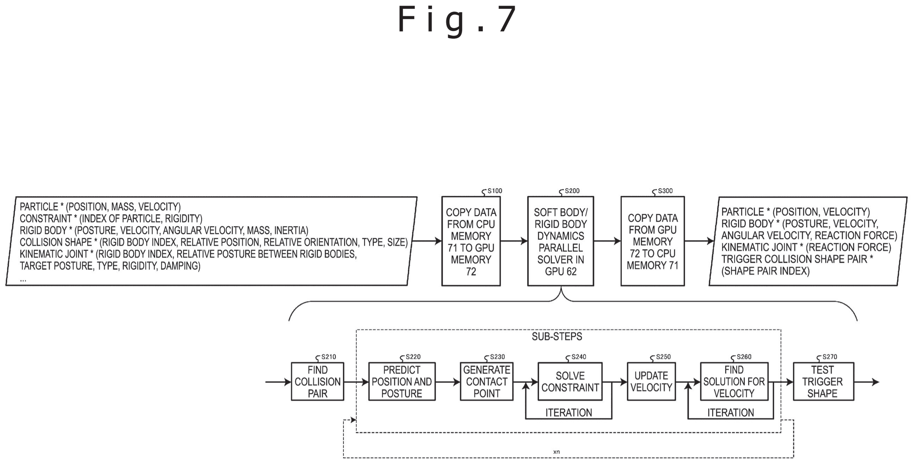

   Fig. 7 is a flowchart illustrating an example of processing (simulation method) executed in the simulator 1.
[Fig. 8]

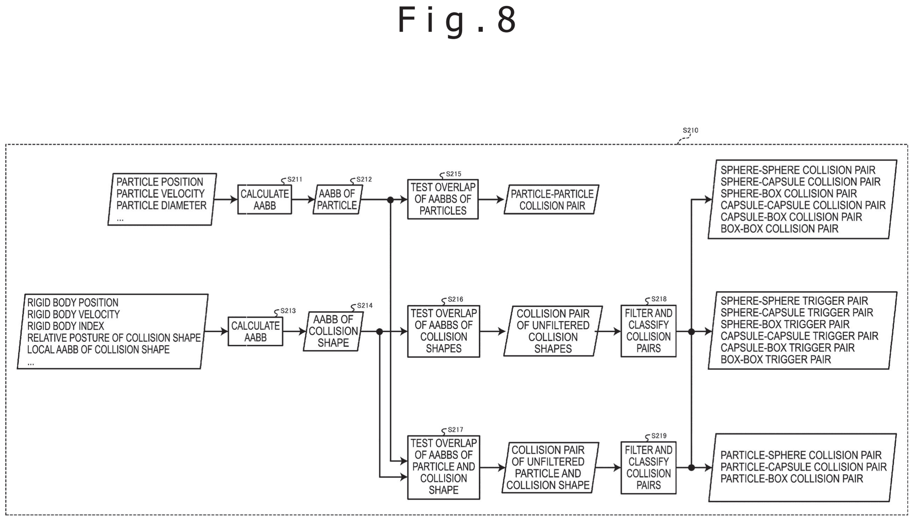

   Fig. 8 is a flowchart illustrating a detailed example of step S210.
[Fig. 9]

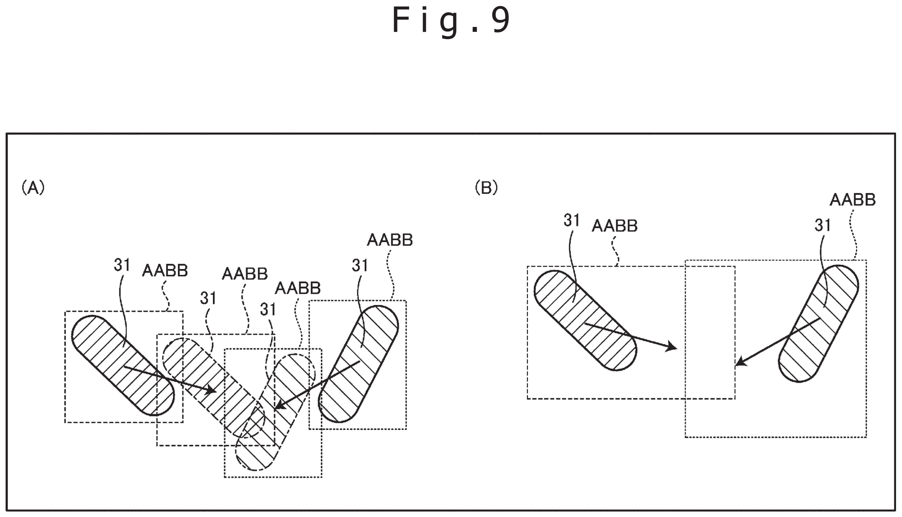

   Fig. 9 is a diagram illustrating examples of finding a collision pair.
[Fig. 10]

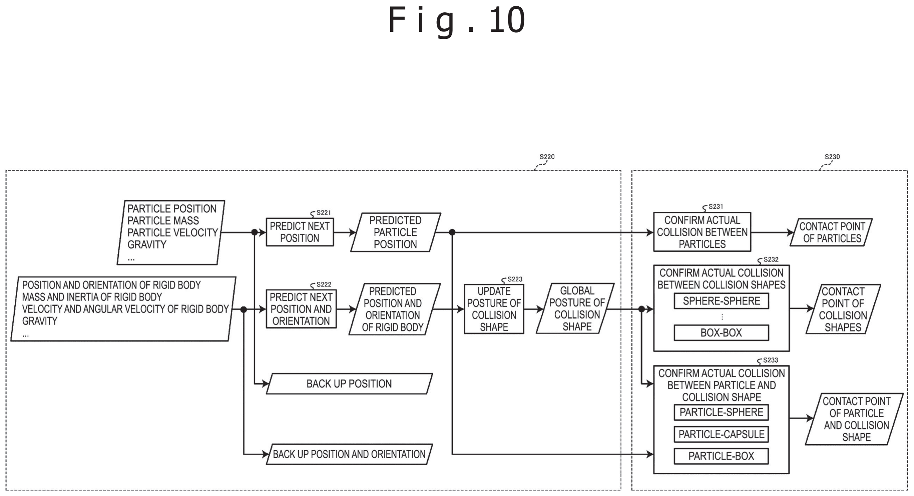

   Fig. 10 is a flowchart illustrating a detailed example of step S220 and step S230.
[Fig. 11]

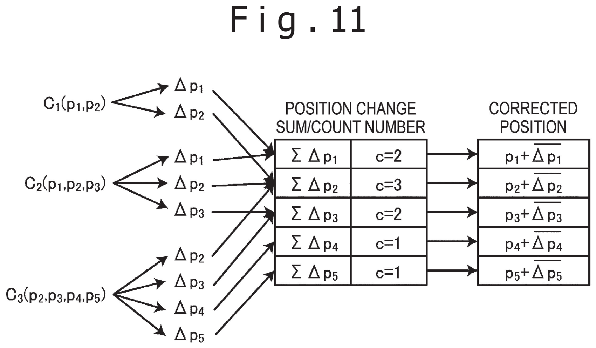

   Fig. 11 is a diagram illustrating an example of a method of solving constraints.
[Fig. 12]

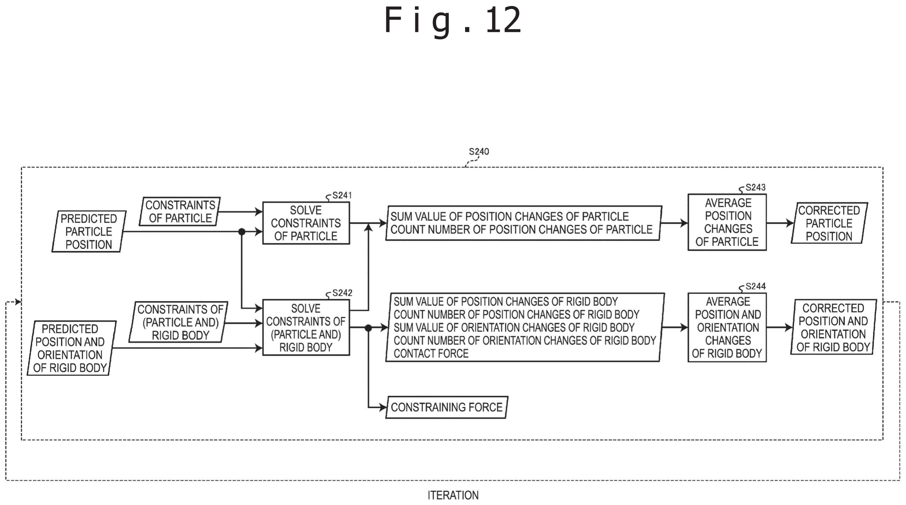

   Fig. 12 is a flowchart illustrating a detailed example of step S240.
[Fig. 13]

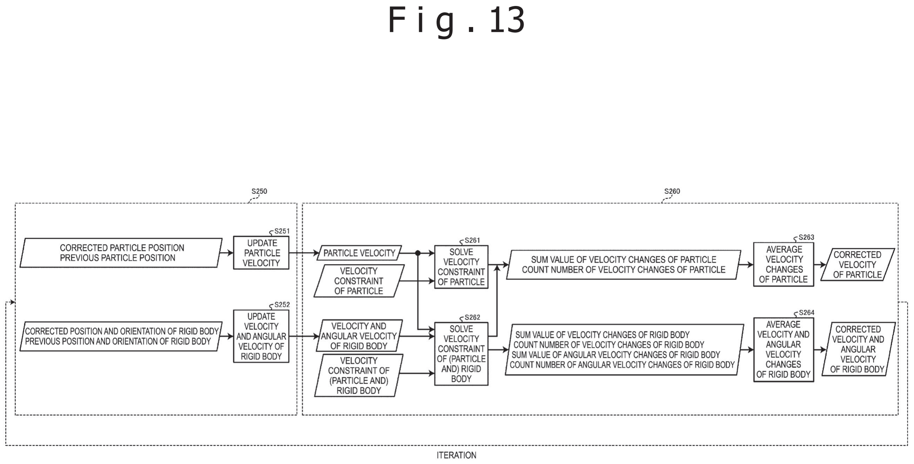

   Fig. 13 is a flowchart illustrating a detailed example of step S250 and step S260.
[Fig. 14]

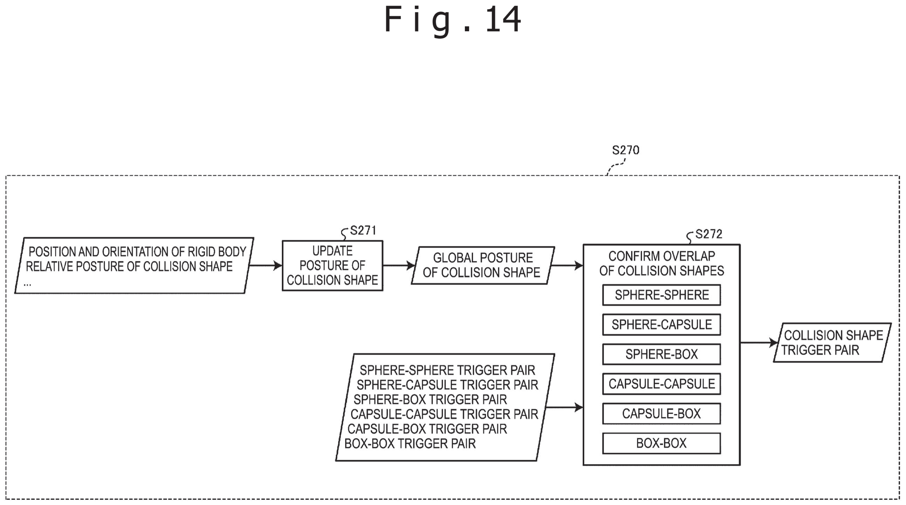

   Fig. 14 is a flowchart illustrating a detailed example of step S270.
[Fig. 15]

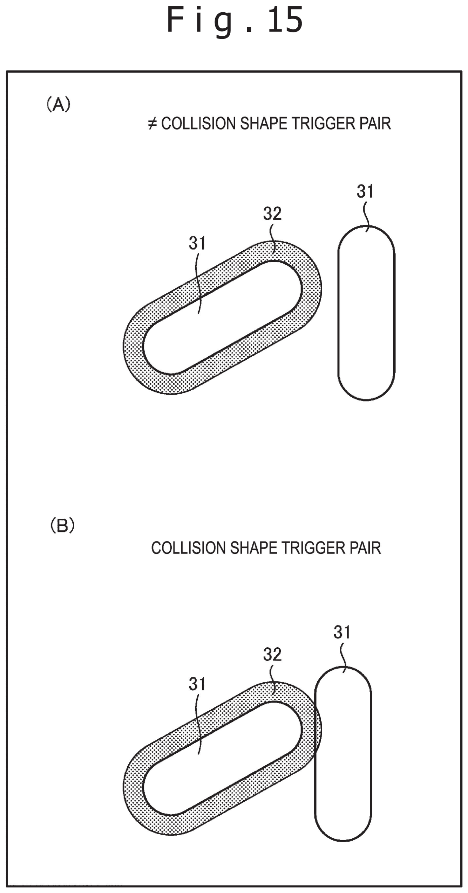

   Fig. 15 is a diagram schematically illustrating a confirmation of an overlap.
[Fig. 16]

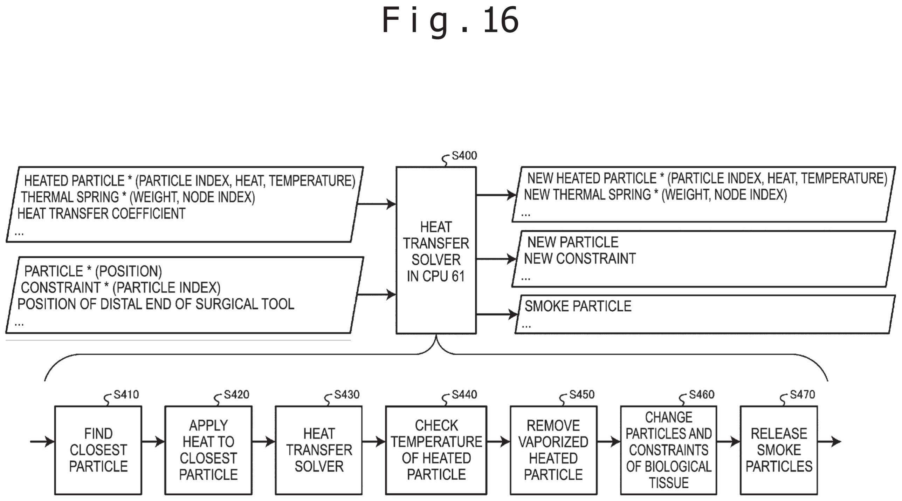

   Fig. 16 is a flowchart illustrating an example of processing (simulation method) executed in the simulator 1.
[Fig. 17]

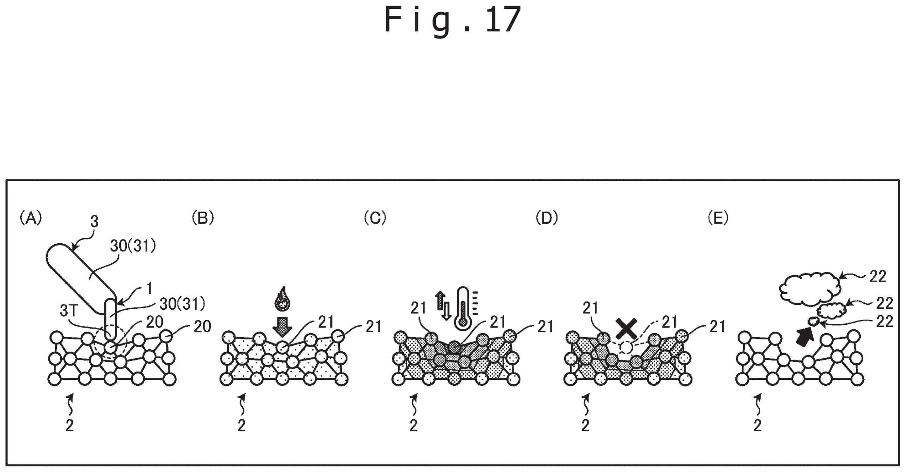

   Fig. 17 is a diagram schematically illustrating step S410 to step S470.
[Fig. 18]

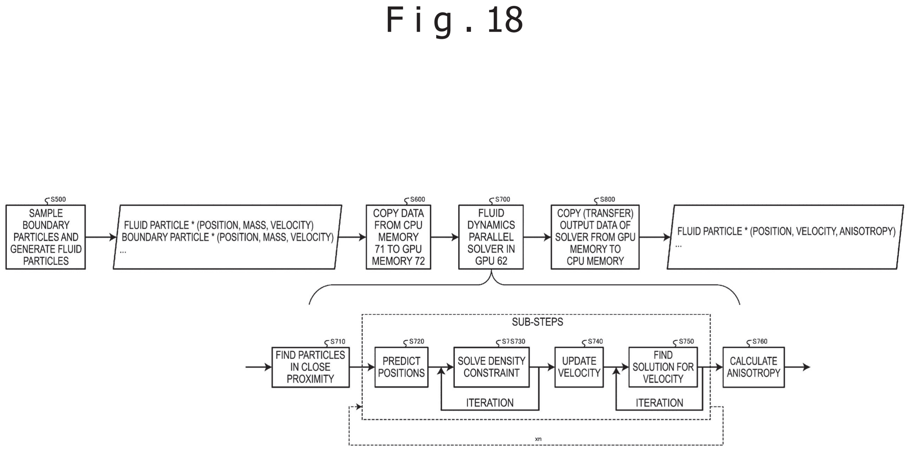

   Fig. 18 is a flowchart illustrating an example of processing (simulation method) executed in the simulator 1.
[Fig. 19]

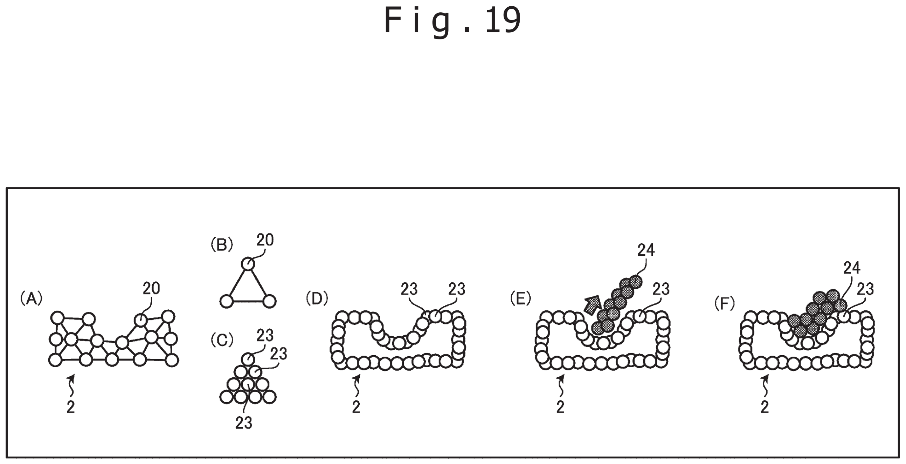

   Fig. 19 is a diagram schematically illustrating step S500.
[Fig. 20]

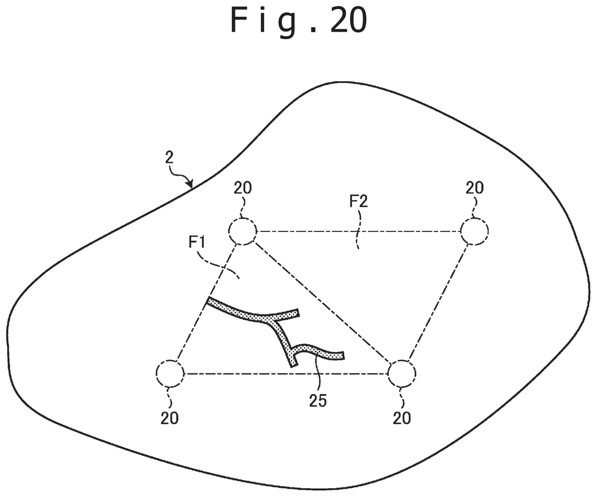

   Fig. 20 is a diagram illustrating an example of cases with and without bleeding.
[Fig. 21]

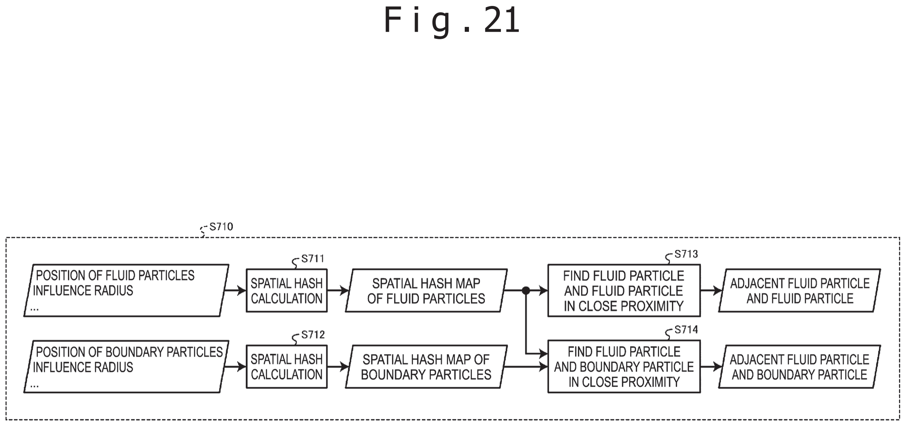

   Fig. 21 is a flowchart illustrating a detailed example of step S710.
[Fig. 22]

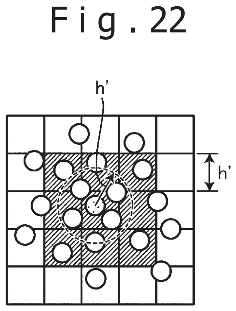

   Fig. 22 is a diagram illustrating an example of calculating using a spatial hash.
[Fig. 23]

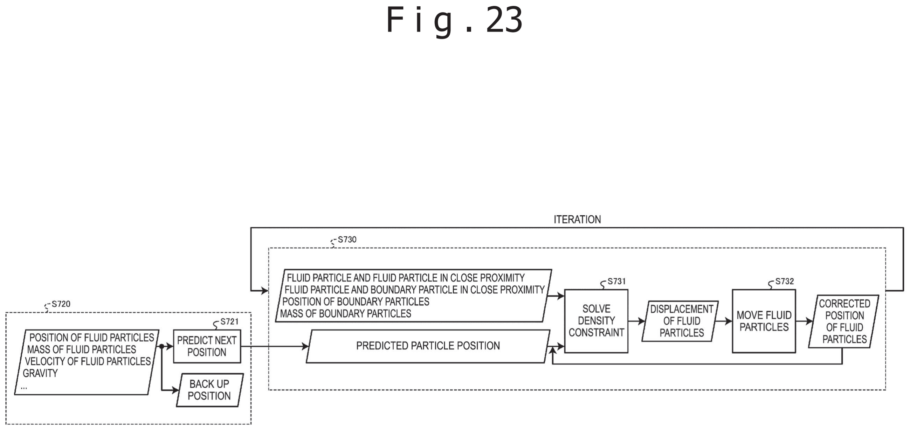

   Fig. 23 is a flowchart illustrating a detailed example of step S720 and step S730.
[Fig. 24]

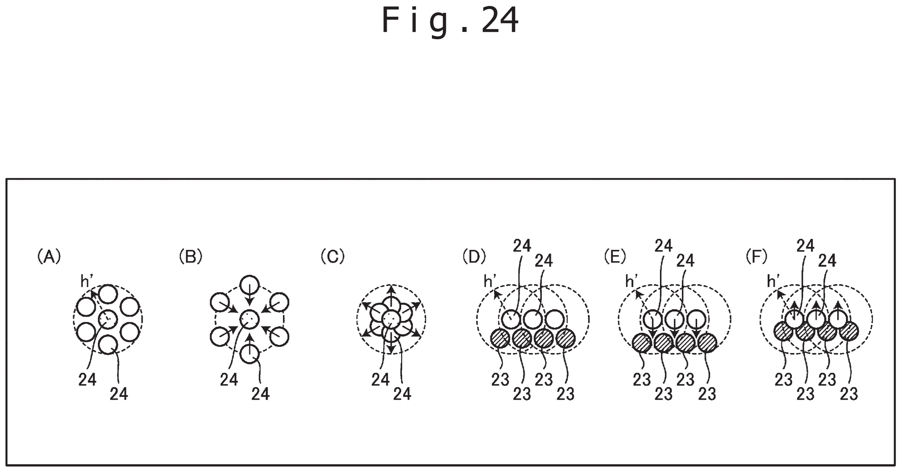

   Fig. 24 is a diagram illustrating an example of a density constraint.
[Fig. 25]

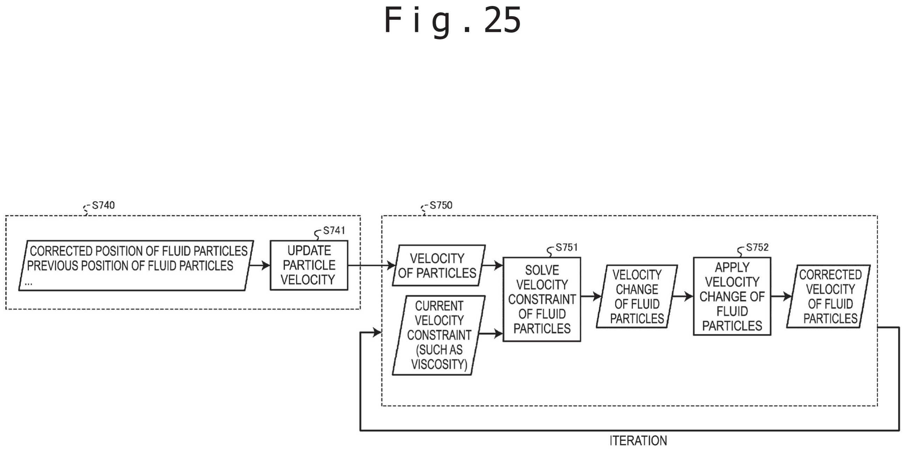

   Fig. 25 is a flowchart illustrating a detailed example of step S740 and step S750.
[Fig. 26]

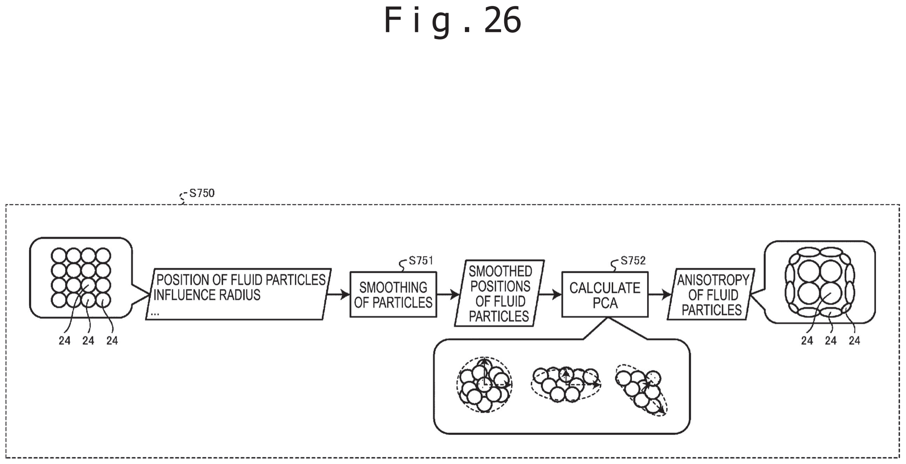

   Fig. 26 is a flowchart illustrating a detailed example of step S760.
[Fig. 27]

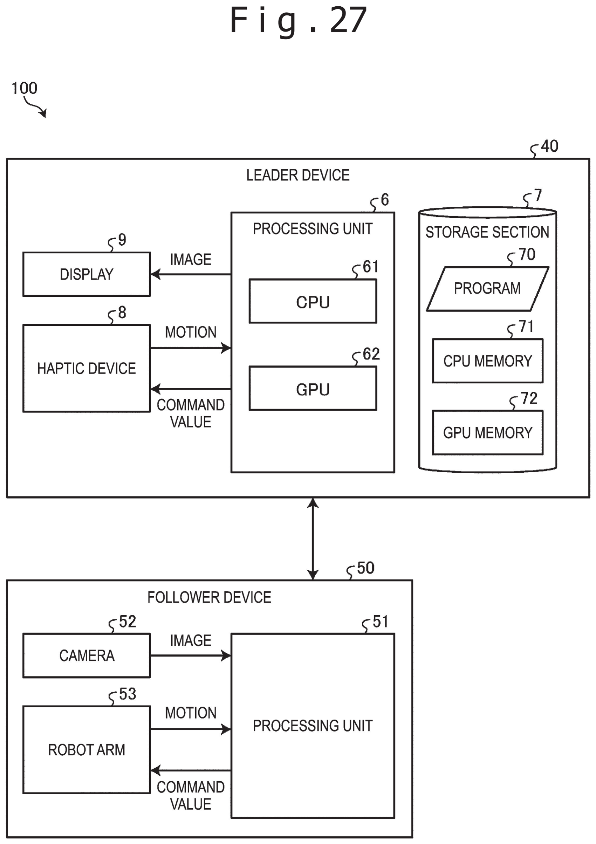

   Fig. 27 is a diagram illustrating a modification.
[Fig. 28]

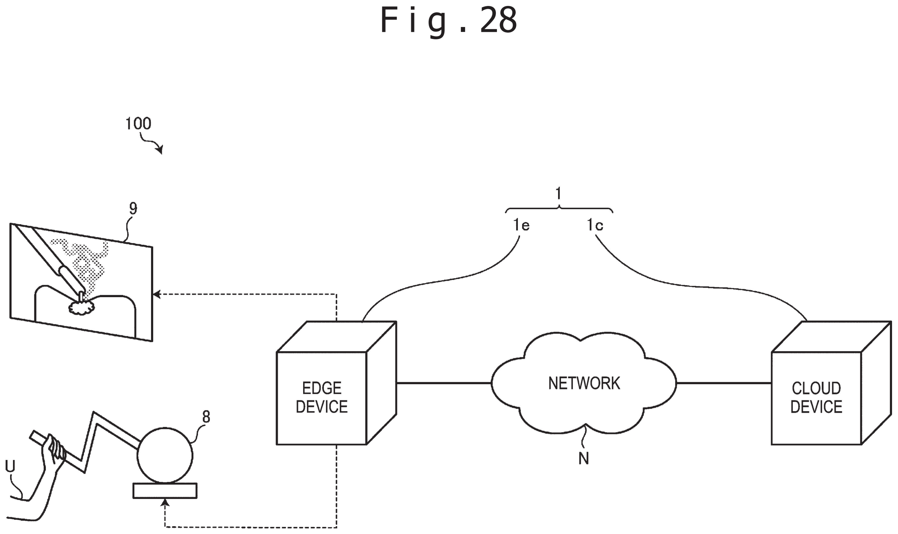

   Fig. 28 is a diagram illustrating a modification.
[Fig. 29]

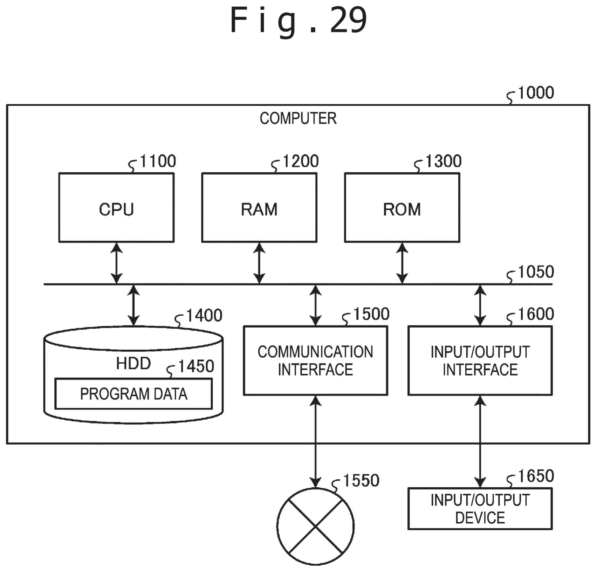

   Fig. 29 is a diagram illustrating an example of a hardware configuration of a device.

### Description of Embodiments

Embodiments of the present disclosure will be described in detail below with reference to the drawings. Note that, in the following embodiments, same elements are denoted by the same reference numerals, and redundant descriptions may be omitted.

The present disclosure will be described in the order of the items below.
0. Introduction
1. Embodiment
   1.1. Modeling of Biological Tissue and Surgical Tool, and Dynamics Simulation
   1.2 Heat Transfer Simulation and Simulation of Smoke Release
   1.3 Fluid Simulation
2. Modifications
3. Example of Hardware Configuration
4. Summary

### 0. Introduction

In a surgical simulation technique in the related art, graphical effects (also referred to as visual effects) such as bleeding and smoke release that may occur when a heated surgical tool such as an electric scalpel is used, are omitted or reduced to very simple effects. This is because, in order to obtain such graphical effects, it is necessary to use different simulation models and algorithms, resulting in high computational costs, and further, it is not easy to use such different simulation models and algorithms in combination. Rendering of realistic blood and smoke also generally involves computational costs.

According to the disclosed technique, for example, in order to simulate a surgery using a heated surgical tool such as an electric scalpel, the heated surgical tool is modeled as an energy device that causes heating in a biological tissue, and the heating in the biological tissue by the energy device, heat propagation in the biological tissue, and the like are simulated. Bleeding is simulated by using Position Based Fluids (PBF) dynamics. The smoke is modeled by using smoke particles. Those are rendered and represented realistically.

In one embodiment, a Central Processing Unit (CPU) and a Graphics Processing Unit (GPU) are used in combination. A real-time simulation is provided that includes cutting or the like of a biological tissue with a surgical tool, and bleeding, smoke release, or the like that may result from the cutting.

### 1. Embodiment

Fig. 1 is a diagram illustrating an example of a schematic configuration of a simulator system 100 according to an embodiment. The simulator system 100 includes a simulator 1, a haptic device 8, and a display 9. A user of the simulator system 100 is referred to as a user U. In Fig. 1, only a hand portion of the user U is schematically illustrated.

The simulator 1 executes a simulation according to a simulation scenario 10. One example of a simulation is a surgical simulation, which is depicted in Fig. 1. The simulation scenario 10 includes several virtual objects relating to a surgery. In Fig. 1, a soft body object, a rigid body object, a robot arm 4, and a camera 5 are illustrated as examples of the objects. Note that the soft body object and the rigid body object are simply referred to as an object when no particular distinction is made.

One example of the soft body object is a biological tissue such as an organ, and is referred to and illustrated in the drawings as a biological tissue 2. An example of the rigid body object is a surgical tool, which is referred to and illustrated in the drawings as a surgical tool 3. Examples of the surgical tool 3 include a scalpel and forceps. The scalpel may be a heated surgical tool such as an electric scalpel and an ultrasonic scalpel, that is, an energy device. Unless otherwise specified, the surgical tool 3 includes an energy device. The surgical tool 3 may, as appropriate, be read as "energy device", as long as no contradiction arises.

The robot arm 4 is a surgical robot arm supporting the camera 5. The camera 5 is a camera used for surgery, and picks up an image of a surgical field including the biological tissue 2 and the surgical tool 3. A field of view of the camera 5 is referred to and illustrated in the drawings as a field of view 5f. Note that the "image" may be interpreted to include "video", and "image" and "video" may, as appropriate, be read for each other, as long as no contradiction arises.

The haptic device 8 (tactile device) is an inputting apparatus that receives an operation of the surgical tool 3. The haptic device 8 is operated by the user U touching the haptic device 8 with a hand, for example, in order to move the surgical tool 3. When the user U operates the haptic device 8 to move the surgical tool 3, the surgical tool 3 treats the biological tissue 2, for example, pinches or cuts the biological tissue 2, and thus the surgical simulation proceeds. The simulation is designed so that a position of the biological tissue 2 changes or an orientation of the biological tissue 2 changes when the surgical tool 3 collides with (is in contact with) the biological tissue 2. A force (also referred to as a contact force or a reaction force) generated by the interaction at that time is reflected in the operation of the haptic device 8 and is fed back to the user U. Note that "collide" and "contact" may, as appropriate, be read for each other, as long as no contradiction arises.

The display 9 displays an image in the field of view 5f of the camera 5 described above, that is, an image including at least a part of the biological tissue 2 and the surgical tool 3. The image also includes a simulation result described below, for example, bleeding or smoke release (described below) that may be caused by the treatment of the biological tissue 2 with the surgical tool 3. The user U operates the haptic device 8 while viewing the image displayed on the display 9. The position, the angle, and the like of the camera 5 may also be operated by the user U. An operation unit of the camera 5 may be incorporated in the haptic device 8 or may be provided separately from the haptic device 8. The camera 5 may be operated automatically. For example, the function of a control unit that automatically operates the camera 5 may be included in the simulation scenario 10.

Fig. 2 is a diagram illustrating an example of a functional block of the simulator 1. The simulator 1 includes a processing unit 6 and a storage section 7.

The processing unit 6 includes at least one processor. In Fig. 2, a CPU 61 and a GPU 62 are illustrated as examples of processors.

The storage section 7 stores information used in the simulator 1. A program 70 is illustrated as an example of the information stored in the storage section 7. The program 70 is a simulation program (software, an application program) for causing a computer to function as the simulator 1. The storage section 7 includes a CPU memory 71 and a GPU memory 72. The CPU memory 71 is a memory utilized when the CPU 61 executes various types of processing. The GPU memory 72 is a memory utilized when the GPU 62 executes various types of processing.

The haptic device 8 receives an input of a motion via the processing unit 6. The motion is information including a position, a posture, a force, and the like. The processing unit 6 associates a motion of the surgical tool 3 with the motion input via the haptic device 8. The user U operates the surgical tool 3 via the haptic device 8. The processing unit 6 calculates a contact force generated by the collision between the biological tissue 2 and the surgical tool 3. The contact force is transmitted from the processing unit 6 to the haptic device 8 and fed back to the user U via the haptic device 8.

As described above, the simulation scenario 10 includes the camera 5, and an image imaged by the camera 5 is displayed by the display 9. The image imaged by the camera 5 is transmitted from the processing unit 6 to the display 9 and displayed.

Fig. 3 is a diagram illustrating an example of roles of the CPU 61 and the GPU 62 in the processing unit 6.

The CPU 61 models the biological tissue 2 and the surgical tool 3 in accordance with the simulation scenario 10. The CPU 61 models the biological tissue 2 by using a plurality of particles 20, which will be described in detail below. The CPU 61 models the surgical tool 3 by using the rigid body 30 having zero or one or more collision shapes 31. The surgical tool 3 may include a plurality of rigid bodies 30, and in this case, the rigid bodies 30 may be connected to each other by kinematic joints J. A distal end (or a distal end portion) of the surgical tool 3 is referred to and illustrated in the drawings as a distal end 3T.

Various types of data (simulation data) relating to the biological tissue 2 and the surgical tool 3 modeled by the CPU 61 are copied (transferred) from the CPU 61 to the GPU 62, more specifically, from the CPU memory 71 to the GPU memory 72. The simulation data may be arranged in an array, as described below.

The GPU 62 calculates a change in positions of the particles 20 in the biological tissue 2 and a change in a position and a change in an orientation of the rigid body 30 in the surgical tool 3, which are caused by the collision between the modeled biological tissue 2 and surgical tool 3, so as to satisfy a given constraint. A solver for solving the constraint is implemented in the GPU 62 (soft body/rigid body dynamics parallel solver).

The solver performs various types of calculations using data relating to the particles 20, the constraint, the rigid body 30, the collision shape 31, the kinematic joint J, and the like as input data, which will be described in detail below. As a result, various types of data such as data of the particles 20, the rigid body 30, the kinematic joint J, and a trigger shape (described below) are output as output data. The output data is copied back from the GPU 62 to the CPU 61, and more specifically, from the GPU memory 72 to the CPU memory 71.

As described above, the surgical tool 3 includes an energy device such as an electric scalpel. During the treatment of the biological tissue 2 by the surgical tool 3, when the surgical tool 3 is in contact with the biological tissue 2, the biological tissue 2 is heated, and the biological tissue 2 is cut or the like.

The processing unit 6 simulates at least one of bleeding and smoke release from the biological tissue 2, based on the bleeding and smoke release caused by the treatment of the biological tissue 2 with the surgical tool 3, more specifically, the heating in the biological tissue 2 caused by the surgical tool 3 and the heat propagation in the biological tissue 2. Here, an overview will be described with reference to Fig. 3, and details will be described below. In the simulation described below, the surgical tool 3 (energy device) is regarded as a heat emitter, and due to heat transfer from the surgical tool 3 to the biological tissue 2, heating or the like in the biological tissue 2 is assumed to occur.

In the simulation, heat (thermal energy) is applied to the particles 20 that are in thermal contact with the surgical tool 3 among the particles 20 in the biological tissue 2, and the temperature of the particles 20 rises. The particle 20 to which heat is applied is referred to and illustrated in the drawings as a heated particle 21.

In order to simulate smoke release, the CPU 61 removes, from the biological tissue 2, a particle 20 of which temperature reaches a vaporization temperature, that is, the heated particle 21 in an overheated state, from among the plurality of particles 20. The vaporization temperature may be set freely, and is 100°C, for example. Together with the heated particle 21, a polyhedral element connected thereto (for example, a tetrahedral element described below) may also be removed.

In order to simulate smoke release, the CPU 61 uses smoke particles 22 to model smoke released from a portion of the biological tissue 2 from which the heated particle 21 of which temperature reaches the vaporization temperature is removed. The smoke particles 22 may be rendered by using a known graphics engine, such as, for example, Unreal Engine (trademark name), so that a realistic graphical effect of smoke is obtained.

The simulation of bleeding is executed in the GPU 62. For this purpose, the data (simulation data) including the biological tissue 2 from which the heated particle 21 is removed is copied from the CPU memory 71 to the GPU memory 72.

In order to simulate bleeding, the GPU 62 uses a plurality of fluid particles 24 to model blood flowing out of a portion of the biological tissue 2 from which the heated particle 21 of which temperature reaches the vaporization temperature is removed. The GPU 62 calculates positions, velocity changes, and the like of the plurality of fluid particles 24 so as to satisfy a density constraint (density conservation constraint) of the plurality of fluid particles 24. A solver for solving the density constraint is implemented in the GPU 62 (fluid dynamics parallel solver). The density constraint includes, for example, a constraint by which the plurality of fluid particles 24 maintain a rest density.

More specifically, prior to the processing by the GPU 62, the CPU 61 samples a plurality of boundary particles 23 located on the surface of the biological tissue 2 more densely than the plurality of fluid particles 24 contained in the biological tissue 2. Data including the sampled particles is copied from the CPU memory 71 to the GPU memory 72 as described above. The GPU 62 calculates the positions of the plurality of fluid particles 24, so as to satisfy the density constraints of the plurality of fluid particles 24 and the plurality of boundary particles 23. For example, the density constraint includes a constraint by which, of the plurality of fluid particles 24, fluid particles 24 that are in close proximity to the boundary particles 23 (for example, located within an influence radius) maintain a rest distance from the boundary particles 23.

The GPU 62 calculates the velocity change of the plurality of fluid particles 24 so as to satisfy a velocity constraint of the plurality of fluid particles 24. Examples of the velocity constraint include a velocity constraint due to viscosity, a velocity constraint due to vorticity, a velocity constraint due to adhesion, a velocity constraint due to absorption, a velocity constraint due to friction, a velocity constraint due to cohesion, and a velocity constraint due to surface tension.

Data indicating the results of the above-described calculations by the GPU 62, for example, data including the position of each of the fluid particles 24 and the like, are copied back from the GPU memory 72 to the CPU memory 71.

The CPU 61 transmits, to the display 9, data of an image including the blood modeled by the plurality of fluid particles 24 and the smoke modeled by the smoke particles 22 described above. The blood and the smoke may be rendered by a known graphics engine and then displayed. Realistic graphics of bleeding and smoke release during surgery can be displayed via the display 9.

The above description of Fig. 3 gives a general overview of the simulation. A specific procedure of the simulation will be described below.

### 1.1. Modeling of Biological Tissue and Surgical Tool, and Dynamics Simulation

The modeling of the biological tissue 2 and the surgical tool 3 by the CPU 61 and the simulation of the soft body/rigid body dynamics proceed, based on an extended position-based dynamics (XPBD) method. First, the CPU 61 models the biological tissue 2 by using the plurality of particles 20, and models the surgical tool 3 by using the rigid body 30 having zero or one or more collision shapes 31. The particles 20 and the rigid body 30 will be described by referring also to Figs. 4 and 5.

Fig. 4 is a diagram illustrating an example of one of the particles 20 and the rigid body 30. Coordinates common to the particle 20 and the rigid body 30 (that is, the biological tissue 2 and the surgical tool 3 including the particle 20 and the rigid body 30, respectively) are referred to as global coordinates C_{global}.

The particle 20 is schematically illustrated in the drawing as a sphere having a constant radius r to simplify understanding. The particle 20 may be a point particle. The particle 20 is described by using various types of physical quantities (physical characteristics). In Fig. 4, a mass m, a position p, and a velocity v are illustrated as examples of physical quantities describing the particle 20.

The rigid body 30 is also described by using various types of physical quantities. In Fig. 4, a mass m, a position p, an orientation q, a velocity v, an angular velocity ω, and inertia I are illustrated as examples of physical quantities describing the rigid body 30. The position and velocity of the rigid body 30 may be a center position and a center velocity of the rigid body 30.

If the particle 20 and the rigid body 30 are treated as static bodies, the mass of the particle 20 and the rigid body 30 may be set to zero. When the rotation of the rigid body 30 is fixed, the inertia I of the rigid body 30 may be set to zero.

The particles 20 may collide with each other, and the rigid bodies 30 may also collide with each other. Furthermore, the particles 20 and the rigid bodies 30 may collide with each other. In a collision involving the rigid body 30, the collision shape 31 of the rigid body 30 is taken into consideration. The rigid body 30 is attached with zero, or one or more collision shapes 31. By combining a plurality of collision shapes 31, various collision shapes can generally be attached to the rigid body 30.

When the rigid body 30 has a plurality of collision shapes 31, the collision shapes 31 may have basic shapes (geometric shapes), sizes, postures, and the like different from each other. For example, the posture of each of the collision shapes 31 may be a relative posture (relative position and orientation) defined by local coordinates with reference to the center of mass of the rigid body 30, separately from the global coordinates C_{global}.

Fig. 4 illustrates, as examples, three types of collision shapes 31 having different basic shapes. The collision shapes 31 are referred to and illustrated in the drawing as a collision shape 31-1, a collision shape 31-2, and a collision shape 31-3, so as to be distinguished from each other. When the collision shapes are not particularly distinguished from each other, the collision shapes are simply referred to as the collision shape 31.

The basic shape of the collision shape 31-1 is a spherical shape. The collision shape 31 having a spherical shape is also simply referred to as a "sphere".

The basic shape of the collision shape 31-2 is a capsule shape, in other words, a cylindrical shape, an elliptical shape, or the like having hemispherical shapes at both ends. The collision shape 31 having a capsule shape is also simply referred to as a "capsule".

The basic shape of the collision shape 31-3 is a box shape, in other words, a rectangular parallelepiped shape or the like. The collision shape 31 having a box shape is also simply referred to as a "box".

For example, the rigid body 30 is attached with the collision shape 31 having various types of basic shapes as described above, and thus, the shape of the rigid body 30 relating to the collision is formed. The collision shape 31 of the rigid body 30 may collide with the collision shape 31 of another rigid body 30 or one of the particles 20. Note that the rigid body 30 having no collision shape 31 does not collide with another rigid body 30, but in such case, the movement or momentum of the collision shape 31 caused by an external force is also calculated.

The particles 20 and the rigid bodies 30 are described by using an individual array for each physical quantity (each characteristic), and are stored in the storage section 7, more specifically, the CPU memory 71. For example, quaternions for expressing the rotation are used, and the rotation is stored as a vector of four elements qa = {qa_w, qa_x, qa_y, qa_z} (in which qa_w is a real part). The collision shape 31 is stored in association with a list of indices (indices for identifying the rigid bodies 30) indicating to which rigid body 30 the collision shape 31 is attached, a list of flags indicating the types of basic shapes, a list of vectors indicating the sizes of the collision shapes 31, and the like.

For example, a complex biological tissue 2 such as an organ or an articulated surgical tool 3 such as an instrument for a surgical operation is modeled by combining the particles 20 and the rigid bodies 30 under a given constraint. The constraint is an objective function given for the position of the particle 20, the position and orientation (posture) of the rigid body 30, and the like. In the simulation, the position of the particle 20, the position and orientation of the rigid body 30, and the like are corrected so as to minimize errors therein. By setting a flexibility (compliance) parameter, the constraint is given flexibility. By setting the flexibility to zero (by setting rigidity), the constraint becomes very (infinitely) stiff. The constraint may include a constraint in extended position-based dynamics (XPBD). Several examples of constraints are described by referring also to Figs. 5 and 6.

Figs. 5 and 6 are diagrams illustrating examples of constraints. The constraints illustrated in Fig. 5 are inter-particle constraints restricting relative movement between the particles 20. The movement of each particle 20 in a particle cluster (group) is restricted, maintaining the whole group in a constant structure. In Figs. 5(A) to 5(D), a distance constraint, a two-dimensional FEM (FEM 2D) constraint, a three-dimensional FEM (FEM 3D) constraint, and a volume preservation constraint are schematically illustrated as the inter-particle constraints.

As illustrated in Fig. 5(A), the distance constraint is a constraint for two particles 20. For example, the distance constraint may function as a linear spring maintaining a certain distance between the particles 20.

As illustrated in Fig. 5(B), the FEM 2D constraint is a constraint for three particles 20 being located at vertices and forming a triangular element, and is defined as a plane strain tensor of the triangular element. For example, the FEM 2D constraint is constructed (meshed) along a 3D triangular mesh topology, and used to model thin and soft structures such as membranes.

As illustrated in Fig. 5(C), the FEM 3D constraint is a constraint for four particles 20 being located at vertices and forming a tetrahedral element, and is defined as a strain tensor of the tetrahedral element. The FEM 3D constraint is constructed along an input tetrahedral mesh, for example, and is used for modeling the biological tissue 2 having a volume, such as an organ.

As illustrated in Fig. 5(D), the volume preservation constraint is a constraint on a plurality of particles 20 forming a closed triangle mesh in which the plurality of particles 20 are located at vertices. The volume preservation constraint maintains the volume of the closed triangle mesh constant. The volume preservation constraint is used for modeling a balloon-shaped biological tissue 2 such as a gallbladder which is an elastic membrane filled with an incompressible fluid, for example.

The constraints illustrated in Fig. 6 are inter-rigid-body constraints that restrict relative movement between the rigid bodies 30 and are a constraint between the rigid body and the particle that restricts relative movement between the rigid bodies 30 and the particles 20. For the sake of simplicity, the rigid bodies 30 are depicted as cubes, and the collision shapes 31 thereof are not illustrated in the drawing.

The inter-rigid-body constraints restrict the relative movement between the rigid bodies 30, as with a kinematic constraint. As the inter-rigid-body constraints, a fixed joint constraint, a distance joint constraint, a hinge or revolute joint constraint, a slider or prismatic joint constraint, a cylindrical joint constraint, a spherical joint constraint, and a 6 Degree of Freedom (DOF) joint constraint are schematically illustrated in Figs. 6(A) to 6(G).

As illustrated in Fig. 6(A), the fixed joint constraint maintains the relative posture of the rigid bodies 30. For example, the fixed joint constraint is used to join two rigid bodies 30 so that the rigid bodies 30 behave as one rigid body.

As illustrated in Fig. 6(B), the distance joint constraint maintains the distance between attachment points of the rigid bodies 30. The two rigid bodies 30 move as if coupled by a linear spring. The distance joint constraint is used to elastically attach two rigid bodies 30, for example.

As illustrated in Fig. 6(C), the hinge or revolute joint constraint restricts the movement of the rigid bodies 30 to one rotational degree of freedom (1DOF). The rigid bodies 30 cannot perform translational movement, but can rotate about an axis. The hinge or revolute joint is driven according to a target angle and acts as a 1DOF revolute spring. The hinge or revolute joint constraint is used, for example, to model an articulated portion in a robot mechanism or the like, or to model opening and closing operations of a gripping portion of forceps (for example, the surgical tool 3 in Figs. 1 and 3).

As illustrated in Fig. 6(D), the slider or prismatic joint constraint restricts the movement of the rigid bodies 30 to one linear DOF. The rigid bodies 30 cannot rotate, but can slide along one axis. The slider or prismatic joint is driven according to a targeted linear displacement and acts as a linear spring. The slider or prismatic joint constraint is used to simulate a linear actuator in a mechanism, for example.

As illustrated in Fig. 6(E), the cylindrical joint constraint is a combination of the hinge or revolute joint constraint and the slider or prismatic joint constraint. The rigid bodies 30 can slide or rotate about an axis. The cylindrical joint constraint is used to model a mechanism more complex than a mechanism modeled by the hinge or revolute joint alone or the slider or prismatic joint alone, for example.

As illustrated in Fig. 6(F), the spherical joint constraint limits the linear motion of the rigid body 30 to an attachment point. The rigid body 30 can rotate with three degrees of freedom (3DOF). The spherical joint is driven according to a target alignment and acts as a 3DOF revolute spring. The spherical joint constraint is used to model an articulated portion such as a shoulder.

As illustrated in Fig. 6(G), the 6DOF joint constraint is a combination of a linear spring and an angular spring. The 6DOF joint acts as an elastic 6DOF attachment portion between the rigid bodies 30. The 6DOF joint constraint is used to cause the rigid body 30 to follow a target posture by, for example, attaching to a stationary rigid body 30 having no collision shape 31. For example, as described above, a virtual tool (the surgical tool 3) can be moved by the movement input from the haptic device 8.

The constraint between the rigid body and the particle illustrated in Fig. 6(H) is a special kinematic joint constraint used to join the rigid body 30 to one or a plurality of the particles 20. For example, the joint constraint between the rigid body and the particle is used to maintain the relative positions of the particle 20 and the rigid body 30 to which the particle 20 is attached, and to combine the rigid body 30 and a soft body formed of the particles 20.

The various types of constraints described above are described by using, for example, an individual array for each physical quantity of the constraint target, and are stored in the storage section 7, more specifically, in the CPU memory 71. For example, a list of pairs of indices indicating connected particles 20, a list of rest distances, a list of flexibility values, and the like, are defined to represent the distance between the particles 20. These lists may vary depending on the constraints. For example, the 2D FEM constraint uses indices of three particles 20 per constraint, and the 3D FEM constraint uses indices of four particles 20.

Returning back to Fig. 1, the simulation proceeds, based on simulation data including information relating to the modeled particles 20, the rigid bodies 30, the constraints, and the like, as described above, for example. One objective of the simulation is to calculate, so as to satisfy given constraints, a change in the position of the particles 20, a change in the position of the rigid bodies 30, and a change in the orientation of the rigid bodies 30, which are caused by the collision between the modeled objects.

In an XPBD in the related art, a sequential iterative solver is used to sequentially process the constraints, and after each constraint is processed, the position and the orientation are immediately updated. However, in this way, there is a problem in that the processing becomes slow, as the number of constraints increases. Other dynamics engines are based on heterogeneous computing architectures, for example. That is, the portion of the solver that fundamentally serves as a time series is implemented in the CPU, and a parallelizable algorithm is implemented in the GPU. In contrast, in the present embodiment, a complete parallel solver is implemented in the GPU 62 for both the particles 20 and the rigid bodies 30. This will be described with reference to Fig. 7 and subsequent drawings.

The dynamics solver for a soft body such as the biological tissue 2 and a rigid body such as the surgical tool 3 will be described with reference to Figs. 7 to 15.

Fig. 7 is a flowchart illustrating an example of processing (simulation method) executed in the simulator 1. Simulation data as illustrated on the left side in Fig. 7 is prepared, and stored in the CPU memory 71.

In step S100, the processing unit 6 copies (transfers) data from the CPU memory 71 to the GPU memory 72. As described above, the data is simulation data including data relating to the biological tissue 2 and the surgical tool 3 modeled by the CPU memory 71, data relating to the constraints, and the like, and serves as input data to be input to the GPU 62. Examples of the input data are described below, and in Fig. 7, the input data is indicated before step S100.
- Data relating to the particle 20, for example, data indicating the particle 20 and the position, the mass, and the velocity thereof.
- Data relating to the constraint, for example, data indicating the content of the constraint, an index of the particle 20 to which the constraint is applied (an index for identifying the particle 20), and the rigidity given by the constraint.
- Data relating to the rigid body 30, for example, data indicating the rigid body 30 and the posture, the velocity, the angular velocity, the mass, and the inertia thereof.
- Data relating to the collision shape 31, for example, data indicating an index of the rigid body 30 to which the collision shape 31 is attached, the relative position, the relative orientation, the type of the basic shape, and the size of the collision shape 31.
- Data relating to the kinematic joint, for example, data indicating an index of the rigid bodies 30 connected by the kinematic joint, the relative postures of the rigid bodies 30, the target posture, the type, the rigidity, and the damping of the rigid bodies 30.

The data may be described as an array (for example, a two-dimensional array (a plane array)), so that the data can be copied efficiently without reformatting the data. This can contribute to improving the efficiency of the processing.

In step S200, the solver implemented in the GPU 62, more specifically the soft body/rigid body dynamics parallel solver, executes various types of internal processing necessary for the simulation. The internal processing corresponds to processing for one time step Δt in the simulation. Data indicating the results obtained by the processing by the solver is stored in the GPU memory 72.

In step S300, the data is copied from the GPU memory 72 to the CPU memory 71. The data can also be referred to as output data of the GPU 62. Examples of the output data are as follows, and in Fig. 7, the output data is indicated after step S300.
- Data relating to the particle 20, for example, data indicating the particle 20 and the position and the velocity thereof.
- Data relating to the rigid body 30, for example, data indicating the rigid body 30 and the posture (the position and the orientation), the velocity, the angular velocity, and the reaction force thereof.
- Data relating to the kinematic joint, for example, data indicating the kinematic joint and the reaction force thereof.
- Data relating to a trigger collision shape pair (described below), for example, data indicating a trigger collision shape pair and data indicating an index of the shape pair thereof (an index for identifying the trigger collision shape pair).

The processing in step S100, step S200, and step S300 described above is repeatedly executed for each time step Δt, and the simulation proceeds. The processing by the solver in the GPU 62 in step S200 will be further described. The solver in the GPU 62 may be simply referred to as a solver. "Solver" may, as appropriate, be read as "the GPU 62", as long as no contradiction arises.

In Fig. 7, the processing in step S200 is illustrated in a further subdivided manner in step S210 to step S270.

In step S210, the solver finds a collision pair. This will be described by referring also to Figs. 8 and 9.

Fig. 8 is a flowchart illustrating a detailed example of step S210. In step S211 to step S214, the solver finds an overlapping (superimposed) pair of particles 20, an overlapping pair of collision shapes 31, and an overlapping pair of the particle 20 and the collision shape 31, as well as pairs that may overlap. For this purpose, an Axis Aligned Bounding Box (AABB) is calculated. Note that, unless otherwise specified, the AABB is an AABB in global coordinates.

Specifically, the solver calculates an AABB of the particle 20 and an AABB of the collision shape 31 in a parallel manner. For example, the AABB of the particle 20 is calculated based on data such as the position, the velocity, and the diameter of the particle 20 (step S211 and step S212). The AABB of the collision shape 31 is calculated based on, for example, the position, the velocity, and the index of the rigid body 30, the relative posture of the collision shape 31, the AABB in local coordinates, and the like (step S213 and step S214). This will be described by referring also to Fig. 9.

Fig. 9 is a diagram illustrating an example of finding a collision pair. Velocity vectors of two collision shapes 31 are schematically indicated by arrows.

In the example illustrated in Fig. 9(A), the two collision shapes 31 are predicted to overlap during the time step Δt, as indicated virtually by dot-dash lines. The velocity of each of the collision shapes 31 is referred to as a velocity v, and each of the AABBs is pushed by a distance (= vΔt) obtained by multiplying the velocity v by the time step Δt. If the movement of the collision shape 31 is a small rotation within the time step Δt, the velocity of the collision shape 31 is the same as the velocity of the rigid body 30 to which the collision shape 31 is attached. As illustrated in Fig. 9(B), by pushing the AABBs using the velocity vectors, it is possible to predict an overlap of the collision shapes 31 and further predict a collision that occurs within one time step Δt.

In one embodiment, there may be a special collision shape 31 that is not dependent on the collision. Such a collision shape 31 may be freely tagged with a "trigger" flag. The overlap is also predicted for the special collision shape 31, and the result may be announced at the end of the time step Δt. This function is useful for implementing, as an event, a test for a trigger shape described below.

Returning back to Fig. 8, in step S215 to step S217, the solver finds a collision pair of particles 20, a collision pair of collision shapes 31, and a collision pair of the particle 20 and the collision shape 31 that are assumed for an actual overlap. In step S215, all possible AABB pairs of particles 20 are tested, and as a result, a list of collision pairs of particles 20 is obtained. In step S216, all possible AABB pairs of collision shapes 31 are tested. In step S217, all possible pairs of particles 20 and collision shapes 31 are tested. The three types of processing in step S215 to step S217 are executed in a parallel manner. That is, the solver finds a collision pair by testing, in a parallel manner, an overlap between the particles 20, an overlap between the collision shapes 31, and an overlap between the particles 20 and the collision shapes 31.

In step S216 and step S217 described above, a collision pair including the collision shape 31 is found. In the subsequent step S218 and step S219, the solver filters and classifies, to create a list, all the collision pairs (collision pairs before filtering) found in step S216 and step S217 according to each basic shape of the collision shape 31, as well as according to whether a trigger flag is present or the like.

Specifically, collision pairs of the collision shapes 31 among the collision pairs without the trigger flag are filtered. The collision pairs are classified, to create a list, into a collision pair of spheres, a collision pair of a sphere and a capsule, a collision pair of a sphere and a box, a collision pair of capsules, a collision pair of a capsule and a box, or a collision pair of boxes.

Furthermore, collision pairs of the particle 20 and the collision shape 31 are filtered. The collision pairs are classified, to create a list, into a collision pair of the particle 20 and a sphere, a collision pair of the particle 20 and a capsule, or a collision pair of the particle 20 and a box.

Moreover, collision pairs with a trigger flag (collision pairs of collision shapes 31) are filtered. The collision pairs are classified, to create a list, into a trigger pair of spheres, a trigger pair of a sphere and a capsule, a trigger pair of a sphere and a box, a trigger pair of capsules, a trigger pair of a capsule and a box, or a trigger pair of boxes.

The above-described creation of a list is effective in efficiently performing processing in a parallel manner when the same type of collision shapes 31 (such as spheres) collide with each other, for example. The two types of processing in step S218 and step S219 are also executed in a parallel manner. As a result of the processing in step S216 to step S219, a list of collision pairs of collision shapes 31, trigger pairs of collision shapes 31, and collision pairs of the particle 20 and the collision shape 31 is obtained.

A change in the position of the particle 20, a change in the position of the rigid body 30, and a change in the orientation of the rigid body 30 included in the collision pairs found here are later calculated by the solver.

Returning back to Fig. 7, the processing in step S220 to step S260 is executed as an inner loop in which one time step Δt in the simulation is divided into n substeps (sub-time steps h = Δt/n). The processing in step S220 to step S260 is repeatedly executed for the predetermined number of iterations (n times).

In step S220 and step S230, the solver predicts a position and a posture and generates a contact point. The contact point is a constraint between the particle 20 and the rigid body 30, and causes the particle 20 and the rigid body 30 to move so as not to penetrate each other. This will be described by referring also to Fig. 10.

Fig. 10 is a flowchart illustrating a detailed example of step S220 and step S230.

In step S221, the solver predicts (calculates) the next position of the particle 20, based on the position, the mass, the velocity, the gravity, and the like of the particle 20. In this calculation, for example, the velocity, the (influence of) gravity during a sub-time step h, and the like of the particle 20 currently being measured are combined. The position of the particle 20 before the prediction is temporarily stored in a backup, for example, the GPU memory 72, so as to be utilized for subsequently calculating the velocity of the particle 20.

In step S222, the solver predicts the next position and orientation (posture) of the rigid body 30, based on the position, the orientation, the mass, the inertia, the velocity, the angular velocity, the weight, and the like of the rigid body 30. In this calculation, for example, the angular velocity, the gravity during the sub-time step h, and the like of the particle 20 currently being measured are combined. The next position and orientation of the rigid body 30 before the prediction are backed up, so as to be utilized for subsequently calculating the velocity of the rigid body 30.

In step S223, the solver updates the posture of the collision shape 31. The posture of the collision shape 31 of the rigid body 30 is updated, so that the rigid body 30 takes the position, the orientation, and the like calculated previously in step S222. An updated global posture of the collision shape 31 is obtained.

After the particle 20 shifts to the next position or the collision shape 31 shifts to the next posture as described above, the solver, in step S231 to step S233, confirms (that) an actual collision between the particle 20 and the collision shape 31 (occurs) and generates a contact point. The contact point is a special constraint generated separately from the collision, and is generated in each sub-step for moving the particle 20 and the rigid body 30. Note that the possible collision pair is previously found in step S210 (Figs. 7 and 8), and for the sake of efficiency, the collision is confirmed only for the found collision pair.

In step S231, the solver confirms an actual collision between the particles 20. The solver generates a contact point of the particles 20 relating to the confirmed collision.

In step S232 and step S233, the solver confirms a collision including the collision shape 31 and generates a contact point. In step S232, the solver confirms an actual collision with the collision shapes 31 and generates a contact point of the collision shapes 31 relating to the confirmed collision. In step S233, the solver confirms an actual collision of the particle 20 and the collision shape 31 and generates a contact point of the particle 20 and the collision shape 31 relating to the confirmed collision. In this example, both in step S232 and in step S233, the solver confirms the actual collisions in a parallel manner for each basic shape (sphere, capsule, box) of the collision shape 31.

A change in the position of the particle 20, a change in the position of the rigid body 30, and a change in the orientation of the rigid body 30 included in the collision confirmed (found) here are later calculated by the solver.

A contact force (a reaction force) generated by the contact between objects is also calculated. For example, in the contact between the biological tissue 2 and the surgical tool 3, the forces acting on all the particles 20 colliding with the collision shape 31 of the surgical tool 3 are calculated in step S240, in which a constraint described below is solved, and the forces are accumulated in the rigid body 30 as the contact force. The sum of the contact forces in the respective rigid bodies 30 is the contact force of the entire surgical tool 3.

Returning back to Fig. 7, in step S240, the solver solves a given constraint. The solver calculates a change in the position of the particle 20, a change in the position of the rigid body 30, a change in the orientation of the rigid body 30, and the like, so as to satisfy the constraint. The change calculated here may be a change caused by a collision between objects (the biological tissue 2 and the surgical tool 3). The solver corrects the position of the particle 20, the position and orientation of the rigid body 30, and the like, based on the calculated change in the position of the particle 20, the position and orientation of the rigid body 30, and the like.

The constraint affects a certain number of the particles 20 or the rigid bodies 30, depending on the type of the constraint. When a plurality of constraints are given, for the sake of a more efficient calculation, the constraints may be grouped into the same type of constraints, such as constraints on the position of the particle 20, constraints on the position of the rigid body 30, and constraints on the orientation of the rigid body 30, and the constraints may be solved in a parallel manner. The solver calculates, in a parallel manner, a change in the position of the particle 20, a change in the position of the rigid body 30, a change in the orientation of the rigid body 30, and the like for each of the same type of constraints, so as to satisfy the plurality of given constraints.

Unless otherwise specified, the Jacobi method is used as a method of solving the constraints. This method is advantageous for parallel processing. This will be described by referring also to Figs. 11 and 12.

Fig. 11 is a diagram illustrating an example of a method of solving constraints. A constraint C₁, a constraint C₂, and a constraint C₃ are illustrated as examples of the plurality of constraints to be processed in a parallel manner. These constraints are all positional constraints. The constraint C₁ is a constraint on a position p₁ and a position p₂, and is indicated in the drawing as C₁(p₁, p₂). The constraint C₂ is a constraint on the position p₁, the position p₂, and a position p₃, and is indicated in the drawing as C₂(p₁, p₂, p₃). The constraint C₃ is a constraint on the position p₂, the position p₃, a position p₄, and a position p₅, and is indicated in the drawing as C₃(p₂, p₃, p₄, p₅).

The changes in the positions p₁ to p₅, which are calculated so as to satisfy the constraints respectively, are referred to as position changes Δp₁ to Δp₅. The solver accumulates the calculated position changes Δp₁ to Δp₅ in separate buffers and tracks the number of constraints that affect each of the position changes. Specifically, the buffer is initialized to zero, and the solver uses atomic addition to sum the position changes from the same position calculated so as to satisfy each of the different constraints. The solver counts the number of the position changes, in other words, the number of the constraints (a count number c) used for calculating the position changes. The solver averages the summed positions by the count number and adds the averaged position to the current position, to calculate the latest position (a corrected position).

The above-described method is an example of a method of calculating the positions of the particle 20 and the rigid body 30, and a similar method may be used to calculate the orientation (or the rotation) of the rigid body 30. When the constraint is a kinematic joint constraint of the rigid body 30 or a contact point of the collision shape 31, constraining forces or contact forces obtained as a calculation result may also be each accumulated in a buffer.

Fig. 12 is a flowchart illustrating a detailed example of step S240.

In step S241, the solver solves the constraints on the particle 20, based on constraints given to the particle 20 (for example, FEM-based constraints, contacts, and the like) and predicted positions of the particle. As described above with reference to Fig. 11, the sum value of the position changes of the particle 20 and the count number are obtained.

In step S242, the solver solves the constraints of the rigid body 30 (or the particle 20 and the rigid body 30), based on the constraints (for example, a kinematic joint constraint, contact, and the like) given to the rigid body 30 (or the particle 20 and the rigid body 30) and the predicted position and orientation of the rigid body. The sum values of the position changes and the orientation changes of the rigid body 30, the count number, and the contact force are obtained. A constraining force is also obtained.

In step S243, the solver averages the position changes of the particle 20. As described above with reference to Fig. 11, the sum value of the position changes of the particle 20 is averaged by the count number. The solver adds the averaged position change of the particle 20 to the current position of the particle 20, to calculate the corrected (next) position of the particle 20.

In step S244, the solver averages the position changes and the orientation changes of the rigid body 30. The sum values of the position changes and the orientation changes of the rigid body 30 are averaged by the count number. The solver adds the averaged position change and orientation change of the rigid body 30 to the current position and orientation of the rigid body 30, to calculate the corrected (next) position and orientation of the rigid body 30.

Since performing the above-described averaging only once does not necessarily yield a correct solution, the processing in step S241 to step S244 are repeatedly executed for a fixed number of iterations (position iterations).

Returning back to Fig. 7, in step S250 and step S260, the solver updates the velocity and finds a solution for the updated velocity. For example, the next velocities of the particle 20 and the rigid body 30 are calculated in a parallel manner, and the velocities are corrected so as to satisfy the velocity constraint. In the case of the rigid body 30, the angular velocity is also calculated and corrected. This will be described by referring also to Fig. 13.

Fig. 13 is a flowchart illustrating a detailed example of step S250 and step S260.

In step S251, the solver updates the velocity of the particle 20, based on the corrected position of the particle 20 and the previous position of the particle 20. The corrected position of the particle 20 described here is a position of the particle 20 after the correction in the previous step 240, and corresponds to the latest position of the particle 20. The previous position of the particle 20 is the position of the particle 20 backed up in the previous step S220.

In step S252, the solver updates the velocity and the angular velocity of the rigid body 30, based on the corrected position and orientation of the rigid body 30 and the previous position and orientation of the rigid body 30. The corrected position and orientation of the rigid body 30 described here are the position and orientation of the rigid body 30 after the correction in the previous step S240, and correspond to the latest position and orientation of the rigid body 30. The previous position and orientation of the rigid body 30 are the position and orientation of the rigid body 30 backed up in the previous step S220.

In step S261, the solver solves the velocity constraint of the particle 20, based on the velocity of the particle 20 and the velocity constraint of the particle 20. For example, the velocity constraint is a constraint on the velocity due to friction (static friction, dynamic friction), contact, damping in a kinematic joint, and the like. The velocity constraint is used to model, for example, friction and rebound (elasticity) of the collision shape 31 and to add damping to the kinematic joint. A method of solving the constraint is similar to the methods described above, and includes calculations such as summing and counting of the velocity changes of the particle 20.

In step S262, the solver solves the velocity constraint of the rigid body 30 (or of the particle 20 and the rigid body 30), based on the velocity and the angular velocity of the rigid body 30 and the velocity constraint of the rigid body 30 (or of the particle 20 and the rigid body 30). A method of solving the constraint is similar to the methods described above, and includes calculations such as summing and counting the velocity changes and the angular velocity changes of the rigid body 30 (or the particle 20 and the rigid body 30).

In step S263, the solver averages the velocity changes of the particle 20. The solver adds the averaged velocity change of the particle 20 to the current velocity of the particle 20, to calculate the corrected (next) velocity of the particle 20.

In step S264, the solver averages the velocity changes and the angular velocity changes of the rigid body 30. The solver adds the averaged velocity change and the averaged angular velocity change of the rigid body 30 to the current velocity and the current angular velocity of the rigid body 30, to calculate the corrected (next) velocity and angular velocity of the rigid body 30.

Since performing the above-described averaging only once does not necessarily yield a correct solution, the processing in step S251 to step S264 is repeatedly executed for a fixed number of iterations (velocity iterations).

Returning back to Fig. 7, after the processing in step S220 to step S260 is repeatedly executed n times as one sub-step processing, the processing proceeds to step S270.

In step S270, the solver tests a trigger shape (described below). The test described here includes an overlap test of the trigger shape. This will be described by referring also to Figs. 14 and 15.

Fig. 14 is a flowchart illustrating a detailed example of step S270.

In step S271, the solver updates the posture of the collision shape 31, based on the position and the orientation of the rigid body 30, the relative posture of the collision shape 31, and the like. A global posture of the collision shape 31 is obtained.

In step S272, the solver confirms an overlap between the collision shapes 31 (for example, finds a collision shape trigger pair described below), based on a global posture of the collision shapes 31 and a trigger pair for each basic shape of the collision shapes 31. The trigger pair is obtained in step S210 described above, and the description thereof will not be repeated. The confirmation of the overlap will be described by referring also to Fig. 15.

Fig. 15 is a diagram schematically illustrating the confirmation of the overlap. The collision shapes 31 illustrated as an example are capsules. A shape that can overlap with the collision shape 31 is referred to and illustrated in the drawing as a trigger shape 32. If there is a trigger shape 32 that actually overlaps with the collision shape 31, the solver finds the pair of the collision shapes 31 as a collision shape trigger pair. In the example illustrated in Fig. 15(A), the trigger shape 32 of one of the collision shapes 31 does not overlap with the other collision shape 31, and no collision shape trigger pair is found. In the example illustrated in Fig. 15(B), the trigger shape 32 of one of the collision shapes 31 overlaps with the other collision shape 31, and the collision shapes 31 are found as a collision shape trigger pair.

Returning back to Fig. 14, in step S262 illustrated as an example, the solver confirms an overlap in a parallel manner for each combination of basic shapes of the collision shapes 31. The index of the confirmed (found) collision shape trigger pair (an index for identifying a collision trigger pair) is added to a list.

Returning back to Fig. 7, after the processing in step S200, that is, the series of processing by the solver on the GPU 62, is completed, data indicating a result of the processing is copied back from the GPU memory 72 to the CPU memory 71, as described above. The resulting data includes a new position of the particle 20, a new position and orientation (posture) and a contact force of the rigid body 30, a kinematic joint constraint, a trigger shape pair, and the like.

The position, the deformation, and the like of the biological tissue 2 are updated, based on the new position and the like of the particle 20. The position, the orientation, the deformation, and the like of the surgical tool 3 are updated, based on the new position, orientation, and the like of the rigid body 30. The display 9 displays a video of them. The contact force is fed back to the user U via the haptic device 8 (tactile feedback). The series of processing is repeated, and the simulation proceeds. A bidirectional dynamics simulation of the biological tissue 2 and the surgical tool 3 is possible.

By employing a fully parallel dynamics solver implemented in the GPU 62, the real-time properties of the simulation and the tactile feedback are improved. The accuracy of the calculation of the deformation of the biological tissue 2, the tactile force, and the like by using the FEM-based constraints is improved, and it is possible to provide a real-time simulation of a more complicated surgical operation scenario or the like.

An interactive simulation (based on bidirectional interaction) of the biological tissue 2 and the surgical tool 3, based on the XPBD method, is possible (integrated dynamics solver). The surgical tool 3 affects the surgical tool 3 or the biological tissue 2, and thus, a more realistic interactive surgical simulation is possible.

The finding (detection) of a collision and the like can also be processed in a parallel manner by the solver mounted on the GPU 62. Therefore, it is possible to process in real time calculations relating to a very large number (for example, several thousands) of particles 20 and rigid bodies 30, and further, calculations relating to a constraint and the like.

The contact force obtained from the interaction between the biological tissue 2 and the surgical tool 3 by the solver is also consistent with the characteristics such as the mass and the rigidity of a virtual object such as the biological tissue 2 and the surgical tool 3. A stable and accurate tactile feedback is obtained.

Referring again to Fig. 3, the biological tissue 2 and the surgical tool 3 are modeled, and the collision, deformation, and the like of the biological tissue 2 and the surgical tool 3 are simulated, in the manner described above, for example.

### 1.2 Heat Transfer Simulation and Simulation of Smoke Release

According to a result of the simulation including the contact between the biological tissue 2 and the surgical tool 3 described above, the CPU 61 simulates heat transfer from the surgical tool 3 to the biological tissue 2 and simulates smoke release. This will be described by referring also to Figs. 16 and 17.

Fig. 16 is a flowchart illustrating an example of processing (simulation method) executed in the simulator 1. Simulation data as illustrated on the left side in Fig. 16 is prepared and stored in the CPU memory 71. Examples of the data are as follows, and in Fig. 16, the data is illustrated before step S400.
- Data relating to the heated particle 21, for example, data indicating the index, heat (thermal energy), and temperature of the heated particle 21
- Data relating to a thermal spring, for example, data indicating the weight and node index of the thermal spring
- Data indicating a heat transfer coefficient
- Data relating to the particles 20 for modeling the biological tissue 2, for example, data indicating positions of the particles 20
- Data relating to a constraint of the particles 20, for example, data indicating the index of the particles 20
- Data indicating the position of the distal end 3T of the surgical tool 3

The thermal spring is a portion (element) connecting heated particles 21 to each other, according to a mesh model of a polyhedral element of the biological tissue 2. The weight of the thermal spring indicates the strength of the connection, and the node index corresponds to a particle index of the connected heated particles 21. The heat transfer coefficient is, for example, a heat transfer coefficient between the heated particles 21, and may define a heat transfer via a thermal spring.

In step S400, the solver implemented in the CPU 61, more specifically a heat transfer solver, executes various types of internal processing necessary for the simulation. Data indicating the results obtained by the processing of the solver is stored in the CPU memory 71. Examples of the data are as follows, and in Fig. 16, the data is illustrated after step S400.
- Data relating to a new heated particle 21, for example, data indicating an index, heat, and temperature of the heated particle 21
- Data relating to a new thermal spring, for example, data indicating a weight and a node index of the thermal spring
- Data indicating the smoke particles 22

After the soft body/rigid body dynamics simulation described above, the position of the distal end 3T of the surgical tool 3 is determined. As described above, the surgical tool 3 is modeled by using the rigid body 30 to which the collision shape 31 is attached.

In Fig. 16, the processing in step S400 is illustrated in a further subdivided manner in step S410 to step S470. This will be described by referring also to Fig. 17.

Fig. 17 is a diagram schematically illustrating step S410 to step S470.

In step S410 of Fig. 16, the solver finds the closest particle 20. Specifically, the closest particle 20 is found as a particle 20 located within a given influence radius from the distal end T of the surgical tool 3. The influence radius is referred to as an influence radius h'. As illustrated in Fig. 17(A), the particle 20 surrounded by a dashed line is found as the closest particle 20. In the drawing, lines connecting the particles 20 schematically indicate the constraints of the particles 20.

In step S420 of Fig. 16, the solver applies heat to the found closest particle 20. Fig. 17(B) illustrates the heated particles 21 obtained by heating the particles 20 of the biological tissue 2. The lines connecting the heated particles 21 schematically indicate a thermal spring. Heat is transferred between the heated particles 21 via the thermal spring (heat propagation in the biological tissue 2). Heat is applied to the particles 20 that are thermally connected to the surgical tool 3 or are thermally connected, via other heated particles 21, to the surgical tool 3 (that interact with the surgical tool 3), resulting in heated particles 21.

In step S430 and step S440 of Fig. 16, the solver solves a heat transfer model to calculate the temperature change of the heated particles 21 and checks (confirms) the temperature of the heated particles 21. As illustrated in Fig. 17(C), the temperature of each of the heated particles 21 is calculated. When the heated particle 21 is closer to the surgical tool 3, the temperature of the heated particle 21 is higher, and the temperature of some of the heated particles 21 may reach the vaporization temperature (for example, 100°C). In this example, the temperature of the heated particle 21 found in the previous drawing, Fig. 17(A), reaches the vaporization temperature.

In step S450 and step S460 of Fig. 16, the solver removes the vaporized heated particle 21 and changes the particles 20 of the biological tissue 2 and constraints of the biological tissue 2. As illustrated by the dot-dash lines in Fig. 17(D), the vaporized heated particle 21 and thermal springs adjacent to the vaporized heated particle 21 are removed. The solver changes (updates) the constraints and the particles 20 contained in the biological tissue 2, so as to obtain a model of the remaining biological tissue 2 obtained by removing the vaporized heated particle 21 and the adjacent thermal springs adjacent.

In step S470 of Fig. 16, the solver changes the heat transfer model in accordance with the change of the particles 20 and the constraints described above, and releases the smoke particles 22 representing the vaporization of the biological tissue 2 caused by the surgical tool 3. As previously described, the smoke particles 22 are rendered by using a known graphics engine such as Unreal Engine.

Referring again to Fig. 3, the heat transfer from the surgical tool 3 to the biological tissue 2 is simulated, and smoke release is simulated, in the manner described above, for example.

### 1.3 Fluid Simulation

Bleeding that may occur due to the cutting of the biological tissue 2 with the above-described surgical tool 3 or the like is also simulated. The simulation proceeds based on fluid dynamics, and more specifically, position based fluid (PBF) dynamics. This will be described by referring to Figs. 18 to 25.

Fig. 18 is a flowchart illustrating an example of processing (simulation method) executed in the simulator 1.

In step S500, the CPU 61 samples the boundary particles 23 and generates the fluid particles 24. This will be described by referring also to Figs. 19 and 20.

Fig. 19 is a diagram schematically illustrating step S500. As illustrated in Fig. 19(A), the biological tissue 2 is modeled. As illustrated in Figs. 19(B) and 19(C), the boundary particles 23 are sampled, from the polyhedral mesh of the plurality of particles 20 located on an outer surface of the biological tissue 2, more densely than the particles 20. This is to prevent the fluid particles 24 from passing between the boundary particles 23. As illustrated in Fig. 19(D), the boundary particles 23 are arranged so as to cover the surface of the biological tissue 2. The internal tissue of the biological tissue 2 is exposed by cutting or the like of the biological tissue 2 with the surgical tool 3, as illustrated in Fig. 19(E), the fluid particles 24 for modeling the blood are generated. As illustrated in Fig. 19(F), the fluid dynamics are solved to simulate the movement of the fluid particles 24, that is, bleeding. For example, the fluid particles 24 move on the boundary particles 23 (on the outer surface of the biological tissue 2).

Cases with and without bleeding, in accordance with a specific portion of the biological tissue 2 cut with the surgical tool 3, may also be simulated. This will be described by referring also to Fig. 20.

Fig. 20 is a diagram illustrating an example of cases with and without bleeding. The biological tissue 2 is rendered with a texture including a blood vessel 25. A facet F1 and a facet F2 are illustrated as examples as facets of the mesh of the polyhedral element containing the particles 20. The facet F1 includes the blood vessel 25. The facet F2, on the other hand, does not include the blood vessel 25.

When a portion, of the biological tissue 2, including the blood vessel 25 is cut, bleeding occurs. In this example, when the facet F1 is cut, the fluid particles 24 for modeling blood are generated. On the other hand, even if a portion, of the biological tissue 2, not including the blood vessel 25 is cut, bleeding does not occur. In this example, the fluid particles 24 are not generated when the facet F2 is cut. Bleeding is only caused when the blood vessel 25 is cut, and thus, the realism of the simulation can be improved.

Returning back to Fig. 18, as a result of the processing in step S500 described above, data relating to the fluid particles 24 and the boundary particles 23, for example, data indicating the positions, the mass, and the velocity of the fluid particles 24 and the boundary particles 23 is obtained.

In step S600, the processing unit 6 copies (transfers) the above-described data from the CPU memory 71 to the GPU memory 72. The data serves as input data to be input to the GPU 62.

In step S700, the solver implemented in the GPU 62, more specifically, the fluid dynamics parallel solver, executes various types of internal processing necessary for the simulation. The internal processing corresponds to processing for one time step Δt in the simulation. Data indicating the results obtained from the processing by the solver is stored in the GPU memory 72.

In step S800, the data is copied from the GPU memory 72 to the CPU memory 71. The data can also be referred to as output data of the GPU 62. Examples of output data include data relating to the fluid particles 24, for example, data indicating the position, velocity, and anisotropy of the fluid particles 24 (described below), and are illustrated after step S800 in Fig. 18.

In Fig. 18, the processing in step S700 is illustrated in a further subdivided manner in step S710 to step S760.

In step S710, the solver finds particles in close proximity. This will be described by referring also to Figs. 21 and 22.

Fig. 21 is a flowchart illustrating a detailed example of step S710. The fluid particles 24 interact with other particles in close proximity, more specifically, with other fluid particles 24 or boundary particles 23 located within the influence radius h'. For each of the fluid particles 24, a list of the boundary particles 23 and fluid particles 24 that are in close proximity is calculated. In this example, a spatial hash algorithm is used to efficiently find the particles in close proximity.

In step S711 and step S712, the solver calculates a spatial hash, based on the position and the influence radius h' of the fluid particles 24 and the boundary particles 23. This will be described by referring also to Fig. 22.

Fig. 22 is a diagram illustrating an example of calculating using a spatial hash. The space containing the particles is divided into a plurality of rectangular grid cells. One grid cell corresponds to a key code in a hash key. The hash key is calculated based on discretized coordinates of the key codes. The particle is inserted into the hash table (associated in the hash table), in accordance with the key of the grid cell including the particle. It is possible to efficiently search for particles in close proximity by checking the particles associated with the same grid cell or adjacent grid cells in the hash table. In the example illustrated in Fig. 22, the size of each of the grid cells is set to the same size as the influence radius h', in terms of efficiency. In a three-dimensional space, it is sufficient to check 27 grid cells. In Fig. 22, nine grid cells among the grid cells are indicated by hatching.

Returning back to Fig. 21, a spatial hash map of the fluid particles 24 and a spatial hash map of the fluid particles 24 are obtained respectively by the processing in step S711 and step S712 described above.

In step S713 and step S714, the solver finds the fluid particles 24 that are in close proximity to each other, and finds the fluid particles 24 and boundary particles 23 that are in close proximity to each other. As described above with reference to Fig. 22, efficiently finding is made possible by using a result of the calculation using the spatial hash.

Returning back to Fig. 18, the processing in step S720 to step S750 is executed as an inner loop in which one time step Δt in the simulation is divided into n substeps (sub-time steps h = Δt/n). The processing in step S720 to step S750 is repeatedly executed for a predetermined number of iterations (n times). Note that the number of iterations described here may be a value different from the number of iterations in step S220 to step S260 in Fig. 7 described above.

In step S720 and step S730, the solver predicts the positions and solves the density constraint. This will be described by referring also to Figs. 23 and 24.

Fig. 23 is a flowchart illustrating a detailed example of step S720 and step S730.

In step S721, the solver predicts the position of the fluid particle 24. Specifically, the solver predicts (calculates) the next position of the fluid particle 24 by integrating the position, the mass, the velocity, the gravity (an example of an external force), and the like of the fluid particle 24 during the sub-time step h. Note that the position of the fluid particle 24 before the prediction is temporarily stored in a backup, for example, in the GPU memory 72, so as to be utilized for subsequently calculating the velocity of the fluid particle 24.

The fluid particles 24 move to the next position, and then, in step S731, the solver solves the density constraint described above. Specifically, the solver calculates the current density, based on the positions and the mass of the fluid particles 24 or the fluid particles 24 and the boundary particles 23 that are in close proximity to each other, the positions and the mass of the boundary particles 23, and the positions of the fluid particles 24 previously predicted in step S721. Furthermore, the solver calculates the displacement of the fluid particles 24 that satisfy the rest density. This will be described by referring also to Fig. 24.

Fig. 24 is a diagram illustrating an example of the density constraint. The density constraint acts to apply (enforce) incompressibility to (on) the fluid particles 24.

As illustrated in Fig. 24(A), a given fluid particle 24, which is the fluid particle 24 in the center in this example, interacts with other fluid particles 24 located within the influence radius h' illustrated by a dashed line. First, the fluid particles 24 uniformly disperse while assuming the rest density. As the fluid particles 24 move and the density changes due to an external force (such as gravity), the density constraint is solved so that each fluid particle 24 is fluid incompressible. For example, as illustrated in Fig. 24(B), when the fluid particles 24 in close proximity to each other scatter and the density becomes smaller than the rest density, the fluid particles 24 in close proximity to each other are pulled back to positions to achieve the rest density. Conversely, as illustrated in Fig. 24(C), when the fluid particles 24 in close proximity to each other are attracted to each other and the density becomes greater than the rest density, the fluid particles 24 in close proximity to each other separate to achieve the rest density.

With regard to the relationship between the fluid particles 24 and the boundary particles 23, as illustrated in Fig. 24(D), the fluid particles 24 at the rest density behave so as to maintain a given rest distance from the boundary particles 23. For example, as illustrated in Fig. 24(E), when the fluid particles 24 separate from the boundary particles 23 within the influence radius h', the fluid particles 24 are pulled back toward the boundary particles 23, so that the rest density is maintained. Conversely, as illustrated in Fig. 24(F), when the fluid particles 24 are pushed toward the boundary particles 23, the fluid particles 24 separate from the fluid particles 24, so that the rest density is maintained.

Returning back to Fig. 23, by the processing in step S731, the displacement of the fluid particles 24 is obtained. In step S732, the solver moves the fluid particles 24 by the calculated displacement of the fluid particles 24. This movement corrects the positions of the fluid particles 24. The positions, of the fluid particles 24, that are corrected are also referred to as the corrected positions of the fluid particles 24.

The solution may not necessarily be settled by executing the processing in step S731 and step S732 only once. Thus, the processing in step S731 and step S732 is executed for a fixed number of iterations (position iterations), based on the corrected positions of the fluid particles 24.

Returning back to Fig. 18, in step S740 and step S750, the solver updates the velocity and finds a solution for the updated velocity. This will be described by referring also to Fig. 25.

Fig. 25 is a flowchart illustrating a detailed example of step S740 and step S750.

In step S741, the solver updates the velocities of the fluid particles 24, based on the corrected positions of the fluid particles 24 and the previous positions of the fluid particles 24. The corrected positions of the fluid particles 24 described here are the positions of the fluid particles 24 after the correction in the previous step 730, and correspond to the latest positions of the fluid particles 24. The previous positions of the fluid particles 24 are the positions of the fluid particles 24 backed up in the previous step S720.

In step S751, the solver calculates the velocity change of the fluid particles 24, based on the updated velocity of the fluid particles 24 and the current velocity constraint. The velocity constraint may be a velocity constraint from a phenomenon, a factor, or the like, which is difficult to model only by the density constraint. As described above, examples of the velocity constraint include constraints due to viscosity, vorticity, adhesion, absorption, friction at the boundary between particles, cohesion, and surface tension. For example, the various types of velocity constraints are solved in a parallel manner (simultaneously) and (vectors of) velocity changes corresponding to each type of the velocity constraints are calculated simultaneously.

In step S752, the solver adds the calculated velocity changes of the boundary particles 23. For example, the velocity changes corresponding to the various types of velocity constraints calculated simultaneously in the previous step S751 are added to the current velocity. Thus, the velocity of the fluid particles 24 is corrected. The velocity, of the fluid particles 24, that is corrected is also referred to as the corrected velocity of the fluid particles 24.

The solution may not necessarily be settled by executing the processing in step S751 and step S752 only once. Thus, the processing in step S751 and step S752 is executed for a fixed number of iterations (velocity iterations), based on the corrected velocities of the fluid particles 24.

Returning back to Fig. 18, in step S760, the solver calculates the anisotropy of the fluid particles 24. The anisotropy of the fluid particles 24 refers to the magnitude, size, and the like (measurements) of a local distribution of the fluid particles 24. For example, the solver calculates the anisotropy of at least some of the fluid particles 24 among the plurality of fluid particles 24 to have an elliptical shape. The calculation will be described by referring also to Fig. 26.

Fig. 26 is a flowchart illustrating a detailed example of step S760.

In step S751, the solver smooths the fluid particles 24, based on the latest positions of the fluid particles 24, the influence radius h', and the like. For example, a smoothing filter is used to reduce nonuniformities in the fluid particles 24. The positions of the fluid particles 24 after smoothing are obtained.

In step S752, the solver executes a Principal Component Analysis (PCA) for each of the fluid particles 24 and the fluid particles 24 in close proximity, to find a centroid and a principal direction of the fluid particles 24. This processing is executed in a parallel manner for the fluid particles 24.

In the example illustrated in Fig. 26, the fluid particles 24 located inside the spherical shape do not have a principal direction. In contrast, the fluid particles 24 located at the surface are flattened so as to have an elliptical shape oriented in a direction of a free fluid surface and have a principal direction.

The anisotropy of the fluid particles 24 is calculated for rendering. The blood is rendered that is modeled by a plurality of fluid particles 24 including the above-described fluid particles 24 having the elliptical shape, to provide more realistic graphical effects.

Referring again to Fig. 3, bleeding is simulated, in the manner described above, for example. The various types of simulations described above are realized by the processing in the processing unit 6, more specifically, by the cooperation of the CPU 61 and the GPU 62. The result of the simulation is fed back to the haptic device 8 or displayed as an image on the display 9. In particular, bleeding and smoke release that may be caused by a treatment such as cutting of the biological tissue 2 with the surgical tool 3 are also simulated, and the rendered blood and smoke are presented to the user U via the display 9. Realistic graphical effects of the bleeding and smoke are provided.

### 2. Modifications

The disclosed technique is not limited to the above-described embodiments. Several modifications will be described.

The simulation described above may be applied to a surgical robot simulation. In the surgical robot simulation, the user U operates, via the haptic device 8, a robot arm supporting a surgical tool. The contact force is fed back to the user U, and thus, it is also possible to evaluate whether the robot arm is operated with an appropriate operation force. More effective training is possible than in a case in which the contact force is not fed back.

In one embodiment, the simulation may be incorporated into a master-slave system. Such a system will be described by referring to Fig. 27.

Fig. 27 is a diagram illustrating a modification. The simulator system 100 includes a leader device 40 and a follower device 50.

The leader device 40 includes the haptic device 8, the display 9, the processing unit 6, and the storage section 7 described above. The leader device 40 is communicable with the follower device 50. A command value for controlling the haptic device 8 is transmitted from the processing unit 6 to the haptic device 8.

The follower device 50 includes a processing unit 51, a camera 52, and a robot arm 53. The processing unit 51 observes the slave environment including a state of a patient, based on a video captured by the camera 52. The slave environment may include non-visual information such as a blood pressure and a heart rate of the patient. The processing unit 51 controls the robot arm 53, in accordance with an operation of the haptic device 8 by the leader device 40. The control is performed by transmitting a command value from the processing unit 51 to the robot arm 53.

The leader device 40 and the follower device 50 function as a master device and a slave device capable of bilateral control. The haptic device 8 is operated by the user U to control, in a master-slave manner, the robot arm 53 of the follower device 50 supporting the surgical tool. The video captured by the camera 52 of the follower device 50 is transmitted from the follower device 50 to the leader device 40 and displayed by the display 9 of the leader device 40. Information on other slave environments is also transmitted from the follower device 50 to the leader device 40 to be presented. The motion (information on such as the position, the posture, and the force) from the haptic device 8 of the leader device 40 is transmitted from the leader device 40 to the follower device 50, and is reflected in the control of the robot arm 53. The motion from the robot arm 53 is transmitted from the follower device 50 to the leader device 40 and fed back to the user via the haptic device 8 of the leader device 40.

The leader device 40 calculates the collision, the deformation (such as a change in the position of the particle 20, and a change in the position and orientation of the rigid body 30), the contact force, and the like of the biological tissue 2 and the surgical tool 3 as described above. In the simulator system 100 illustrated in Fig. 16, a calculation result of the leader device 40 is reflected in the control of the robot arm 53. For example, when the motion from the haptic device 8 of the leader device 40 is transmitted to the follower device 50, the control of the robot arm 53 is restricted to ensure safety.

Specifically, the processing unit 6 of the leader device 40 executes safety determination processing, based on the calculation result of the simulation model. For example, the processing unit 6 determines whether or not the operation of the robot arm 53 of the follower device 50 via the leader device 40 is dangerous. When determining that the operation is dangerous, the processing unit 6 intervenes in the operation of the follower device 50 by the leader device 40. For example, the processing unit 6 corrects the motion transmitted from the leader device 40 to the follower device 50, so that the amount of movement of the robot arm 53 is restricted or the operation force is restricted. The corrected motion is transmitted from the leader device 40 to the follower device 50. The leader device 40 and the follower device 50 are notified of the determination result or the control result by the processing unit 6.

In one embodiment, the functions of the simulator 1 may be provided in a distributed manner. This will be described by referring to Fig. 28.

Fig. 28 is a diagram illustrating a modification. The simulator 1 includes an edge device 1e and a cloud device 1c. The edge device 1e and the cloud device 1c serve as a first information processing apparatus and a second information processing apparatus that cooperate with each other to serve as the simulator 1. The edge device 1e and the cloud device 1c are connected via a network N and transmit and exchange (share) data relating to the simulation.

In one embodiment, the network N may be an all-optical communication network. The cloud device 1c and the edge device 1e are connected by the network N in this case, that is, at least endpoint routers communicate each other entirely by optical communication. Note that the communication after each of the endpoint routers may be electrical communication after photoelectric conversion. By utilizing a broadband all-optical communication network, the cloud device 1c and the edge device 1e are connected with low latency. This is particularly useful for real-time simulation and the like.

The edge device 1e is arranged in an edge region including the haptic device 8 and the display 9. The cloud device 1c is arranged in a cloud region located on the opposite side of the edge region with the network N interposed therebetween. The position of the cloud region may be far from the edge region or may be near the edge region (for example, in the same building or the same room).

The cloud device 1c is arranged in a cloud region CF. The cloud region CF is a region located on the opposite side of an edge region EF with the network N interposed therebetween, and is a region far from the edge region EF, for example.

Any of the functions of the simulator 1 described above may be distributed and arranged in the edge device 1e and the cloud device 1c. As an example, the CPU 61 and the CPU memory 71 may be arranged in the edge device 1e, and the GPU 62 and the GPU memory 72 may be arranged in the cloud device 1c. For example, a portion, of the program 70, corresponding to the edge device 1e may be arranged in the edge device 1e, and a portion, of the program 70, corresponding to the cloud device 1c may be arranged in the cloud device 1c.

According to the above-described simulator 1, a high-performance information processing apparatus arranged in a cloud region can be used as the cloud device 1c, for example. Thus, it is more likely that the simulator 1 can be used as a haptic surgery simulator or the like capable of performing large scale calculations. For example, the simulator 1 can be used for training and preoperative planning of surgeries in which biological tissues (soft organs and the like) are handled, such as a laparoscopic surgery, a thoracic surgery, and a brain surgery. By conducting training while feeling the deformation and a realistic reaction force of the biological tissue, a more accurate prior experience can be performed, and an effect of improving skills and surgery results is expected.

The simulation technique described above may be applied to simulate an endoscopic treatment using a rigid endoscope or a flexible endoscope.

### 3. Example of Hardware Configuration

Fig. 29 is a diagram illustrating an example of a hardware configuration of a device. For example, the simulator 1 is implemented by a computer 1000 illustrated in Fig. 29.

The computer 1000 includes a CPU/GPU 1100, a RAM 1200, a Read Only Memory (ROM) 1300, a Hard Disk Drive (HDD) 1400, a communication interface 1500, and an input/output interface 1600. The units of the computer 1000 are connected to each other via a bus 1050.

The CPU/GPU 1100 operates based on a program stored in the ROM 1300 or the HDD 1400, and controls each unit. For example, the CPU/GPU 1100 loads a program stored in the ROM 1300 or the HDD 1400 onto the RAM 1200, and executes processing corresponding to the various types of programs. An example of the program is the program 70 described above with reference to Fig. 2.

The ROM 1300 stores a boot program such as a Basic Input Output System (BIOS) executed by the CPU/GPU 1100 when the computer 1000 is activated, a program depending on hardware of the computer 1000, and the like.

The HDD 1400 is a computer-readable recording medium that non-transitorily records a program executed by the CPU/GPU 1100, data used by the program, and the like. Specifically, the HDD 1400 is a recording medium recording a program for the information processing method according to the present disclosure, which is an example of program data 1450.

The communication interface 1500 is an interface for the computer 1000 to connect to an external network 1550 (for example, the Internet). For example, the CPU/GPU 1100 receives data from other devices and transmits data generated by the CPU/GPU 1100 to other devices, via the communication interface 1500.

The input/output interface 1600 is an interface for connecting an input/output device 1650 and the computer 1000. For example, the CPU/GPU 1100 receives data from an input device such as a keyboard and mouse, via the input/output interface 1600. Furthermore, the CPU/GPU 1100 transmits data to an output device such as a display, speakers, and a printer, via the input/output interface 1600. For example, the CPU/GPU 1100 receives motion data from the haptic device 8 and transmits a simulated contact force to the haptic device 8, via the input/output interface 1600. The input/output interface 1600 may function as a media interface that reads a program or the like recorded on a predetermined computer-readable recording medium. Examples of the medium include an optical recording medium such as a Digital Versatile Disc (DVD) or a Phase change rewritable Disk (PD), a magneto-optical recording medium such as a Magneto-Optical Disk (MO), a tape medium, a magnetic recording medium, and a semiconductor memory.

When the computer 1000 functions as the simulator 1 described above, the CPU/GPU 1100 of the computer 1000 executes the program loaded onto the RAM 1200 to achieve a function of the processing unit 6. The program may be stored in the HDD 1400. The CPU/GPU 1100 reads the program data 1450 from the HDD 1400 and executes the program data 1450. However, in another example, the CPU/GPU 1100 may acquire the program from another device via the external network 1550.

Each of the above-described constituent elements may be configured by using a general-purpose member, or may be configured by hardware specialized for the function of each constituent element. The configuration can be appropriately changed in accordance with the technical level at the time of implementation.

### 4. Summary

The technique described above is defined as follows, for example. One of the disclosed techniques is the simulator 1. As described with reference to Figs. 1 to 3, Figs. 16 to 26, and the like, the simulator 1 includes the processing unit 6 including at least one processor (for example, the CPU 61 and the GPU 62). The processing unit 6 simulates at least one among bleeding and smoke release from the biological tissue 2, based on the heating in the biological tissue 2 caused by an energy device (the surgical tool 3), and the heat propagation in the biological tissue 2. For example, the energy device is a heated surgical tool such as an electric scalpel and an ultrasonic scalpel, and the heating in the biological tissue 2 is caused by the treatment of the biological tissue 2 by the energy device. With the simulator 1, it is possible to simulate a surgery with bleeding and smoke release.

As described with reference to Figs. 3, 5, 16, 17, and the like, the biological tissue 2 includes the plurality of particles 20, and the processing unit 6 may remove, from the biological tissue 2, the particles 20 (the heated particles 21) of which temperature reaches the vaporization temperature due to heating, among the plurality of particles 20. Bleeding and smoke release caused by heat transfer from the surgical tool 3 to the biological tissue 2 can be simulated in this manner, for example.

As described with reference to Figs. 16 and 17, in order to simulate smoke release, the processing unit 6 may use the smoke particles 22 to model smoke release from a portion of the biological tissue 2 from which the particles 20 (the heated particles 21 in the overheated state) of which temperature reaches the vaporization temperature are removed. Smoke release can be simulated in this manner, for example.

As described with reference to Figs. 18 and 19, in order to simulate bleeding, the processing unit 6 may use the plurality of fluid particles 24 to model blood flowing out of a portion of the biological tissue 2 from which the particles 20 (the heated particles 21) of which temperature reaches the vaporization temperature are removed. Bleeding can be simulated in this manner, for example.

As described with reference to Figs. 18, 23, 24, and the like, the processing unit 6 may calculate the positions of the plurality of fluid particles 24 so as to satisfy the density constraint of the plurality of fluid particles 24. In this case, the density constraint may include a constraint by which the plurality of fluid particles 24 maintain a rest density. The flow of blood can be simulated in this manner, for example.

As described with reference to Figs. 23, 24, and the like, the processing unit 6 may sample the plurality of boundary particles 23 located on the surface of the biological tissue 2 more densely than the plurality of fluid particles 24 included in the biological tissue 2, and calculate the positions of the plurality of fluid particles 24 so as to satisfy the density constraints of the plurality of fluid particles 24 and the plurality of boundary particles 23. In this case, the density constraint may include a constraint by which fluid particles 24, of the plurality of fluid particles 24, in close proximity to the boundary particles 23 maintain a given rest distance from the boundary particles 23. Blood flowing on the surface of the biological tissue 2 can be simulated in this manner, for example.

As described with reference to Figs. 18, 25, and the like, the processing unit 6 may calculate the velocity change of the plurality of fluid particles 24, so as to satisfy the velocity constraint of the plurality of fluid particles 24. The velocity constraint may include at least one of a velocity constraint due to viscosity, a velocity constraint due to vorticity, a velocity constraint due to adhesion, a velocity constraint due to absorption, a velocity constraint due to friction, a velocity constraint due to cohesion, and a velocity constraint due to surface tension. The flow of blood can be simulated in this manner, for example.

As described with reference to Figs. 18, 26, and the like, the processing unit 6 may calculate the anisotropy of the plurality of fluid particles 24 so that at least some of the fluid particles 24 among the plurality of fluid particles 24 have an elliptical shape, and the processing unit 6 may render blood modeled by the plurality of fluid particles 24 including the fluid particles 24 having the elliptical shape. More realistic graphical effects can be provided.

As described with reference to Figs. 18 to 20 and the like, the processing unit 6 may render the biological tissue 2 with textures including a blood vessel, and bleeding may occur when a portion (for example, the facet F1) including the blood vessel 25 in the biological tissue 2 is cut. The realism of the simulation can be improved.

The simulation method described with reference to Figs. 1 to 3, 16 to 26, and the like is also one of the disclosed techniques. The simulation method includes simulating at least one of bleeding and smoke release from the biological tissue 2, based on heating in the biological tissue 2 caused by an energy device (the surgical tool 3), and heat propagation in the biological tissue 2. With such a simulation method as well, it is possible to simulate a surgery with bleeding and smoke release.

The program 70 (simulation program) described with reference to Figs. 1 to 3, 16 to 26, 29, and the like is also one of the disclosed techniques. The program 70 causes the computer 1000 to execute processing of simulating at least one of bleeding and smoke release from a biological tissue, based on the heating in the biological tissue caused by an energy device, and on the heat propagation within the biological tissue. With the above-described program 70 as well, it is possible to simulate a surgery with bleeding and smoke release.

The simulator system 100 described with reference to Figs. 1 to 3, 16 to 26, and the like is also one of the disclosed techniques. The simulator system 100 includes an inputting apparatus (the haptic device 8) that receives an operation of an energy device (the surgical tool 3), and a display device (the display 9) that displays at least one of bleeding and smoke release from the biological tissue 2, which are simulated based on the heating in the biological tissue 2 caused by the energy device, and the heat propagation in the biological tissue 2. With the above-described simulator system 100 as well, it is possible to simulate a surgery with bleeding and smoke release.

Note that the effects described in the present disclosure are merely examples, and are not limited to the disclosed contents. Other effects may be obtained.

Embodiments of the present disclosure have been described above. However, the technical scope of the present disclosure is not limited to the above-described embodiments as such, and various modifications can be made without departing from the gist of the present disclosure. Furthermore, the constituent elements of different embodiments and modifications may be appropriately combined.

Note that the present technology can also have the following configurations.
(1) A simulator including:
   a processing unit including at least one processor, wherein
   the processing unit simulates at least one of bleeding and smoke release from a biological tissue, based on heating in the biological tissue caused by an energy device, and heat propagation in the biological tissue.
(2) The simulator according to (1), wherein
   the energy device is a heated surgical tool, and
   the heating in the biological tissue is caused by a treatment of the biological tissue by using the heated surgical tool.
(3) The simulator according to (1) or (2), wherein the biological tissue includes a plurality of particles, and
   the processing unit removes, from the biological tissue, a particle of which temperature reaches a vaporization temperature due to the heating, among the plurality of particles.
(4) The simulator according to (3), wherein, the processing unit, in order to simulate the smoke release, uses a smoke particle to model smoke released from a portion, of the biological tissue, from which the particle of which temperature reaches the vaporization temperature is removed.
(5) The simulator according to (3) or (4), wherein the processing unit, in order to simulate the bleeding, uses a plurality of fluid particles to model blood flowing out of a portion, of the biological tissue, from which the particle of which temperature reaches the vaporization temperature is removed.
(6) The simulator according to (5), wherein
   the processing unit calculates positions of the plurality of fluid particles to satisfy a density constraint of the plurality of fluid particles.
(7) The simulator according to (6), wherein
   the density constraint includes a constraint by which the plurality of fluid particles maintain a rest density.
(8) The simulator according to (6) or (7), wherein
   the processing unit
   samples a plurality of boundary particles located on a surface of the biological tissue more densely than a plurality of fluid particles included in the biological tissue, and
   calculates positions of the plurality of fluid particles to satisfy a density constraint of the plurality of fluid particles and the plurality of boundary particles.
(9) The simulator according to (8), wherein
   the density constraint includes a constraint by which a fluid particle, of the plurality of fluid particles, in close proximity to the boundary particle maintains a predetermined rest distance from the boundary particle.
(10) The simulator according to any one of (6) to (9), wherein
   the processing unit calculates a velocity change of the plurality of fluid particles to satisfy a velocity constraint of the plurality of fluid particles.
(11) The simulator according to (10), wherein
   the velocity constraint includes at least one of:
   a velocity constraint due to viscosity;
   a velocity constraint due to vorticity;
   a velocity constraint due to adhesion;
   a velocity constraint due to absorption;
   a velocity constraint due to friction;
   a velocity constraint due to cohesion; and
   a velocity constraint due to surface tension.
(12) The simulator according to any one of (5) to (11), wherein
   the processing unit
   calculates an anisotropy of a plurality of fluid particles so that at least some of the plurality of fluid particles have an elliptical shape, and
   renders blood modeled by the plurality of fluid particles including the fluid particles having the elliptical shape.
(13) The simulator according to any one of (1) to (12), wherein
   the processing unit renders the biological tissue with a texture including a blood vessel, and
   the bleeding occurs when a portion, of the biological tissue, including the blood vessel is cut.
(14) A simulation method including
   simulating at least one of bleeding and smoke release from a biological tissue, based on heating in the biological tissue caused by an energy device, and heat propagation in the biological tissue.
(15) A simulation program that causes a computer to execute
   processing of simulating at least one of bleeding and smoke release from a biological tissue, based on heating in the biological tissue caused by an energy device, and heat propagation in the biological tissue.
(16) A simulator system including
   an inputting apparatus configured to receive an operation of an energy device, and
   a display device configured to display at least one of bleeding and smoke release from a biological tissue, which are simulated based on heating in the biological tissue caused by the energy device, and heat propagation in the biological tissue.

### Reference Signs List

100 Simulator system
1 Simulator
1c Cloud device
1e Edge device
2 Biological tissue
20 Particle
21 Heated particle
22 Smoke particle
23 Boundary particle
24 Fluid particle
25 Blood vessel
3 Surgical tool (energy device)
30 Rigid body
31 Collision shape
3T Distal end
40 Leader device
50 Follower device
51 Processing unit
52 Camera
53 Robot arm
6 Processing unit
61 CPU
62 GPU
7 Storage section
70 Program
71 CPU memory
72 GPU memory
8 Haptic device
9 Display
CF Cloud region
EF Edge region
F1 Facet
F2 Facet
N Network
U User
1000 Computer
1050 Bus
1100 CPU/GPU
1200 RAM
1300 ROM
1400 HDD
1450 Program data
1500 Communication interface
1600 Input/output interface
1650 Input/output device

## Claims

1. A simulator comprising:
a processing unit including at least one processor, wherein
the processing unit simulates at least one of bleeding and smoke release from a biological tissue, based on heating in the biological tissue caused by an energy device, and heat propagation in the biological tissue.

2. The simulator according to claim 1, wherein
the energy device is a heated surgical tool, and
the heating in the biological tissue is caused by a treatment of the biological tissue by using the heated surgical tool.

3. The simulator according to claim 1, wherein
the biological tissue includes a plurality of particles, and
the processing unit removes, from the biological tissue, a particle of which temperature reaches a vaporization temperature due to the heating, among the plurality of particles.

4. The simulator according to claim 3, wherein
the processing unit, in order to simulate the smoke release, uses a smoke particle to model smoke released from a portion, of the biological tissue, from which the particle of which temperature reaches the vaporization temperature is removed.

5. The simulator according to claim 3, wherein
the processing unit, in order to simulate the bleeding, uses a plurality of fluid particles to model blood flowing out of a portion, of the biological tissue, from which the particle of which temperature reaches the vaporization temperature is removed.

6. The simulator according to claim 5, wherein the processing unit calculates positions of the plurality of fluid particles to satisfy a density constraint of the plurality of fluid particles.

7. The simulator according to claim 6, wherein
the density constraint includes a constraint by which the plurality of fluid particles maintain a rest density.

8. The simulator according to claim 6, wherein
the processing unit
samples a plurality of boundary particles located on a surface of the biological tissue more densely than a plurality of fluid particles included in the biological tissue; and
calculates positions of the plurality of fluid particles to satisfy a density constraint of the plurality of fluid particles and the plurality of boundary particles.

9. The simulator according to claim 8, wherein the density constraint includes a constraint by which a fluid particle, of the plurality of fluid particles, in close proximity to the boundary particle maintains a predetermined rest distance from the boundary particle.

10. The simulator according to claim 6, wherein
the processing unit calculates a velocity change of the plurality of fluid particles to satisfy a velocity constraint of the plurality of fluid particles.

11. The simulator according to claim 10, wherein
the velocity constraint includes at least one of:
a velocity constraint due to viscosity;
a velocity constraint due to vorticity;
a velocity constraint due to adhesion;
a velocity constraint due to absorption;
a velocity constraint due to friction;
a velocity constraint due to cohesion; and
a velocity constraint due to surface tension.

12. The simulator according to claim 5, wherein
the processing unit
calculates an anisotropy of a plurality of fluid particles so that at least some of the plurality of fluid particles have an elliptical shape; and
renders blood modeled by the plurality of fluid particles including the fluid particles having the elliptical shape.

13. The simulator according to claim 1, wherein
the processing unit renders the biological tissue with a texture including a blood vessel, and
the bleeding occurs when a portion, of the biological tissue, including the blood vessel is cut.

14. A simulation method comprising:
simulating at least one of bleeding and smoke release from a biological tissue, based on heating in the biological tissue caused by an energy device, and heat propagation in the biological tissue.

15. A simulation program that causes a computer to execute
processing of simulating at least one of bleeding and smoke release from a biological tissue, based on heating in the biological tissue caused by an energy device, and heat propagation in the biological tissue.

16. A simulator system comprising:
an inputting apparatus configured to receive an operation of an energy device; and
a display device configured to display at least one of bleeding and smoke release from a biological tissue, the bleeding and the smoke release being simulated based on heating in the biological tissue caused by the energy device, and heat propagation in the biological tissue.
